# EUROPÄISCHE PATENTSCHRIFT

(11) **EP 1 711 473 B1**
(45) Veröffentlichungstag und Bekanntmachung des Hinweises auf die Patenterteilung: **09.01.2008**
(21) Anmeldenummer: 05707002.1
(22) Anmeldetag: 25.01.2005
(51) Int. Cl.: C07D 233/70, A61K 31/4166, A61P 3/04, A61P 3/10, C07D 401/04, C07D 403/08, C07D 401/08

(54) **SUBSTITUIERTE N-CYCLOHEXYLIMIDAZOLINONE MIT MCH-MODULATORISCHER WIRKUNG**
SUBSTITUTED N-CYCLOHEXYLIMIDAZOLINONES HAVING AN MCH-MODULATORY EFFECT
N-CYCLOHEXYLIMIDAZOLINONE SUBSTITUEE A EFFET MODULATEUR MCH

(30) Priorität: 25.01.2004 DE 102004003811
(43) Veröffentlichungstag der Anmeldung: 18.10.2006
(73) Patentinhaber: Sanofi-Aventis Deutschland GmbH, 65929 Frankfurt am Main (DE)
(72) Erfinder: SCHWINK, Lothar, 35260 Stadtallendorf (DE); BOEHME, Thomas, 65428 Rüsselsheim (DE); GOSSEL, Matthias, 65428 Hofheim (DE); STENGELIN, Siegfried, 65817 Eppstein (DE)
(86) Internationale Anmeldenummer: PCT/EP2005/000700
(87) Internationale Veröffentlichungsnummer: WO 2005/070898

(56) Entgegenhaltungen:
- WO-A-03/047568
- WO-A-20/04011438

## Beschreibung

Die Erfindung betrifft substituierte N-Cyclohexylheterozyklen sowie deren physiologisch verträgliche Salze und physiologisch funktionelle Derivate.

Es sind bereits den hier beschriebenen heteroatomsubstituierten Cyclohexylimidazolinonen in ihrer Gesamtstruktur ähnliche Verbindungen mit pharmakologischer Wirkung im Stand der Technik beschrieben. So beschreibt z. B. WO 03/057220 Imidazolon-Derivate (cyclische Harnstoffderivate) mit 5-HT_{2c} Rezeptoraktivität, die zur Behandlung von Störungen des zentralen Nervensystems wie Depressionen oder Angstzustände sowie Magen-Darm-Beschwerden eingesetzt werden können. Diese Verbindungen sind an den zentralen Stickstoffatomen jeweils mit einer aromatischen Gruppe substituiert.

Die nicht vorveröffentlichte prioritätsältere Anmeldung mit dem Aktenzeichen DE 102 33 817.5 (WO 2004/11438) betrifft ebenfalls cyclische Harnstoffderivate, die an den zentralen Stickstoffatomen jeweils mit einer aromatischen Gruppe substituiert sind, wobei eine der aromatischen Gruppen mindestens einen Stickstoff-haltigen Substituenten trägt. Die arylsubstituierten cyclischen Harnstoffderivate weisen eine MCH-modulatorische Wirkung auf. Die Verbindungen eignen sich zum Beispiel als Anorectica.

Des Weiteren sind in EP-A 0 503 548 und EP-A 0 587 134 ähnliche Verbindungen mit pharmakologischer Wirkung beschrieben. EP 0 503 548 und EP 0 587 134 betreffen cyclische Harnstoffderivate, die aggregationshemmende Wirkungen aufweisen, diese Verbindungen enthaltende Arzneimittel und Verfahren zu ihrer Herstellung.

Verbindungen mit MCH-antagonistischer Wirkung zur Behandlung der Obesitas sind im Stand der Technik beschrieben (Beispiele: WO2001021577, WO2003035624, WO2002089729, WO2002006245, WO2002002744, WO2002057233, WO2003045313, WO2003097047, WO2002010146, WO 2003087044).

Der Erfindung lag die Aufgabe zugrunde, Verbindungen zur Verfügung zu stellen, die eine Gewichtsreduktion bei Säugetieren bewirken und die zur Prävention und Behandlung von Obesitas und Diabetes geeignet sind.

Überraschenderweise wurde eine Serie von Verbindungen gefunden, die die Aktivität von MCH-Rezeptoren modulieren. Insbesondere zeichnen sich die Verbindungen durch einen Antagonismus des MCH1R aus.

Die Erfindung betrifft daher Verbindungen der Formel I, 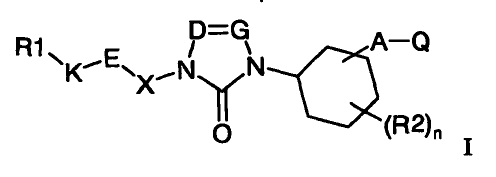 worin bedeuten
- R1: H, (C₁-C₈)-Alkyl, (C₁-C₄)-Alkoxy-(C₁-C₄)-alkyl, (C₃-C₈)-Alkenyl, (C₃-C₈)-Alkinyl, einen 3 bis 10-gliedrigen mono-, bi- oder spirocyclischen Ring welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, NO₂, CN, (C₁-C₆)-Alkyl, O-(C₁-C₈)-Alkyl, S-(C₁-C₈)-Alkyl, (C₁-C₄)-Alkoxy-(C₁-C₄)-alkyl, (C₀-C₈)-Alkylen-aryl, Oxo, CO(R3), CON(R4)(R5), Hydroxy, Hydroxy-(C₁-C₄)-alkyl, COO(R6), N(R7)CO(C₁-C₆)-Alkyl, N(R8)(R9) oder SO₂CH₃;
- R3, R4, R5, R6, R7, R8, R9,: unabhängig voneinander H, (C₁-C₆)-Alkyl;
- K: eine Gruppe der Formel -(CR10R11)_{z}-, worin eine oder mehrere Gruppen - (CR10R11)- durch Z ersetzt sein können, eine Bindung, C≡C, C=C;
- Z: O, CO, N(R59), S, SO, SO₂;
- R10, R11: unabhängig voneinander H, (C₁-C₈)-Alkyl, Hydroxy-(C₁-C₄)-alkyl, Hydroxy, (C₁-C₄)-Alkoxy-(C₁-C₄)-alkyl, wobei R10 und R11 in den z Gruppen jeweils die gleichen oder verschiedene Bedeutungen aufweisen können;
- z: 1, 2, 3, 4, 5, 6;
- R59: H, (C₁-C₈)-Alkyl;
- E: 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, NO₂, CN, OCF₃, Oxo, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)-Alkylen-aryl, O-(C₀-C₈)-Alkylen-aryl, S-Aryl, N(R12)(R13), SO₂-CH₃, COOH, COO-(C₁-C₆)-Alkyl, CON(R14)(R15), N(R16)CO(R17), N(R18)SO₂(R19), CO(R20) tragen und mono- oder bicyclisch sein kann; bevorzugt 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, NO₂, CN, OCF₃, Oxo, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, N(R12)(R13), SO₂-CH₃, N(R16)CO(R17), N(R18)SO₂(R19), CO(R20) tragen und mono- oder bicyclisch sein kann;
- R12, R13, R14, R15, R16, R18: unabhängig voneinander H, (C₁-C₈)-Alkyl;
- oder:
- R12 und R13, R14 und R15: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe N-(C₁-C₆)-Alkyl, Sauerstoff und Schwefel beinhalten kann;
- R17, R19, R20: unabhängig voneinander H, (C₁-C₈)-Alkyl, Aryl;
- X: eine Bindung, eine Gruppe der Formel -(CR21R22)_{y}-, worin eine oder mehrere Gruppen -(CR21R22)- durch Y ersetzt sein können;
- Y: O, CO, N(R23), S, SO, SO₂;
- R21, R22: unabhängig voneinander H, (C₁-C₄)-Alkyl, wobei R21 und R22 in den y Gruppen jeweils die gleichen oder verschiedene Bedeutungen aufweisen können;
- y: 1, 2, 3, 4, 5, 6; bevorzugt 2, 3, 4, 5, 6;
- R23: H, (C₁-C₈)-Alkyl;
- D, G: CH oder N;
- R2: OH, O-(C₁-C₆)-Alkyl, O-(C₁-C₄)-Alkoxy-(C₁-C₄)-alkyl, (C₁-C₆)-Alkyl;
- n: 0, 1, 2, 3, 4;
- Q: N(R24)(R25), einen 3 bis 8-gliedrigen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit (C₀-C₄)-Alkylen-N(R24)(R25), bevorzugt N(R24)(R25); F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃;
- R26, R27, R28, R29, R30: unabhängig voneinander H, (C₁-C₆)-Alkyl ;
- A: eine Gruppe der Formel -(C(R31)(R32))ₘ-, in der 0-2 Glieder ersetzt sein können durch ein Element aus der Gruppe O, S, N(R33), CO, SO₂;
- m: 0, 1, 2, 3, 4, 5;
- R31, R32, R33: unabhängig voneinander H, (C₁-C₆)-Alkyl, Aryl;
- R24, R25: unabhängig voneinander H, (C₁-C₈)-Alkyl, -(CR34R35)ₒ-R36, (C₁-C₄)-Alkoxy-(C₁-C₄)-alkyl, (C₃-C₈)-Akenyl, (C₃-C₈)-Alkinyl, CO-(C₁-C₈)-Alkyl, -CO-(CH₂)ₒ -R36, CO(C(R37)(R38))_{q}N(R39)(R40), CO(C(R41)(R42))ₛO(R43); oder R24 und R25 bilden zusammen mit dem Stickstoffatom, an das sie gebunden sind, einen 4 bis 10-gliedrigen mono-, bi- oder spirocyclischen Ring welcher ausser dem Stickstoffatom 0 bis 4 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, Hydroxy-(C₁-C₄)-alkyl, (C₀-C₈)-Alkylen-aryl, Oxo, CO(R44), CON(R45)(R46), Hydroxy, COO(R47), N(R48)CO(C₁-C₆)-Alkyl, N(R49)(R50) oder SO₂CH₃;
- o: 0, 1, 2, 3, 4, 5, 6;
- q, s: unabhängig voneinander 0, 1, 2, 3, 4;
- R34, R35: unabhängig voneinander H, (C₁-C₈)-Alkyl, Hydroxy-(C₁-C₄)-alkyl, OH, (C₁-C₄)-Alkoxy-(C₁-C₄)-alkyl;
- R37, R38, R39, R40, R41, R42, R43, R44, R45, R46, R47, R48, R49, R50: unabhängig voneinander H, (C₁-C₆)-Alkyl;
- R39 und R40, R45 und R46, R49 und R50: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe N-(C₁-C₆)-Alkyl, Sauerstoff und Schwefel beinhalten kann;
- R36: OH, O-(C₁-C₆)-Alkyl, O-(C₀-C₈)-Alkylen-aryl, CON(R51)(R52), N(R53)(R54), 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, I, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, O-(C₀-C₈)-Alkylen-aryl, (C₀-C₈)-Alkylen-aryl, N(R55)(R56), CO(C₁-C₆)-Alkyl, COO(R57) und S(O)ᵤ (R58) enthalten kann;
- u: 0, 1, 2;
- R51, 52: unabhängig voneinander H, (C₁-C₈)-Alkyl, (C₂-C₆)-Alkenyl, (C₀-C₈)-Alkylen-aryl;
- R53, R54: unabhängig voneinander H, (C₁-C₆)-Alkyl;
- R55, R56: unabhängig voneinander H, (C₁-C₈)-Alkyl;
- R57: H, (C₁-C₈)-Alkyl, (C₂-C₆)-Alkenyl, (C₀-C₈)-Alkylen-aryl;
- R58: (C₁-C₆)-Alkyl, 5-10 gliedriges aromatisches Ringsystem, das 0-2 weitere Heteroatome aus der Gruppe Stickstoff, Sauerstoff und Schwefel enthalten und mit F, Cl, Br, CF₃, NO₂, CN, (C₁-C₈)-Alkyl, O-(C₁-C₈)-Alkyl substituiert sein kann;
sowie deren physiologisch verträgliche Salze.

Bevorzugt weisen die Reste R1 und R2, der Index n und die Gruppen K, E, X, D=G, Q und A unabhängig voneinander die folgenden Bedeutungen auf:
- R1: H, (C₁-C₈)-Alkyl, (C₁-C₄)-Alkoxy-(C₁-C₄)-alkyl, ein 3-8 gliedriger monocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, (C₁-C₆)-Alkyl, O-(C₁-C₈)-Alkyl, (C₁-C₄)-Alkoxy-(C₁-C₄)-alkyl;
bevorzugt (C₁-C₈)-Alkyl, ein 3-7 gliedriger monocyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, (C₁-C₆)-Alkyl, O-(C₁-C₈)-Alkyl;
besonders bevorzugt (C₁-C₆)-Alkyl, (C₃-C₇)-Cycloalkyl und Phenyl.
- K: O, eine Bindung, C≡C, CO, OCH₂, OCH₂CH₂,
bevorzugt O, eine Bindung;
besonders bevorzugt O.
- E: eine 5-6 gliedrige monocyclische bivalente carbo- oder heterocyclische Ringstruktur mit 0-2 Heteroatomen aus der Gruppe N, O und S, die optional Substituenten aus der Gruppe H, F, Cl, Br, Oxo, O-(C₁-C₆)-Alkyl, O-(C₁-C₄)-Alkoxy-(C₁-C₄)-alkyl, (C₁-C₆)-Alkyl tragen kann;
bevorzugt eine para- oder meta-substituierte 6 gliedrige aromatische oder heteroaromatische Ringstruktur, die optional 1-2 Substituenten aus der Gruppe F, Cl, Br, O-(C₁-C₆)-Alkyl, (C₁-C₆)-Alkyl tragen kann;
besonders bevorzugt eine unsubstituierte 1,4-Phenylen- oder 2,5-Pyridyleneinheit.
- X: eine Bindung, CH₂CH₂, CH₂CH₂CH₂, OCH₂CH₂;
bevorzugt eine Bindung, CH₂CH₂;
besonders bevorzugt eine Bindung.
- D=G: CH=CH, CH=N, N=CH;
bevorzugt CH=CH, N=CH;
besonders bevorzugt CH=CH.
- R2: OH, O-(C₁-C₆)-Alkyl, (C₁-C₆)-Alkyl ;
bevorzugt OH, (C₁-C₆)-Alkyl ;
besonders bevorzugt (C₁-C₆)-Alkyl.
- n: 0, 1, 2;
bevorzugt 0, 1;
besonders bevorzugt 0.
- Q: eine Gruppe der Formel N(R24)(R25), ein 3 bis 8-gliedriger Ring mit 0-3 Heteroatomen aus der Gruppe Sauerstoff, Stickstoff und Schwefel, wobei das Ringsystem zusätzlich substituiert sein kann mit N(R24)(R25) F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-Aryl, oxo, CO(R26), CON(R27)(R28), Hydroxy, COO(R29), N(R30)CO(C₁-C₆)-Alkyl, oder SO₂CH₃;
bevorzugt eine Gruppe der Formel N(R24)(R25), ein stickstoffhaltiger 4 bis 8-gliedriger Ring, der außer dem Stickstoffatom noch weitere 0-2 Heteroatomen aus der Gruppe Sauerstoff, Stickstoff und Schwefel enthalten kann und wobei das Ringsystem zusätzlich substituiert sein kann mit N(R24)(R25), F, (C₁-C₆)-Alkyl, O-(C₁-C₈)-Alkyl, (C₁-C₄)-alkoxy-(C₁-C₄)-alkyl, (C₀-C₈)-Alkylen-Aryl, oxo, CO(R26), N(R30)CO(C₁-C₆)-Alkyl, oder SO₂CH₃;
besonders bevorzugt eine Gruppe der Formel N(R24)(R25), ein stickstoffhaltiger 5 bis 8-gliedriger Ring, der außer dem Stickstoffatom noch weitere 0-1 Heteroatomen aus der Gruppe Sauerstoff, Stickstoff und Schwefel enthalten kann und wobei das Ringsystem zusätzlich substituiert sein kann mit N(R24)(R25), (C₁-C₆)-Alkyl, O-(C₁-C₈)-Alkyl, (C₀-C₈)-Alkylen-Aryl, oxo, CO(R26), N(R30)CO(C₁-C₆)-Alkyl, oder SO₂CH₃;
ganz besonders bevorzugt eine Gruppe der Formel N(R24)(R25), ein stickstoffhaltiger 5 bis 8-gliedriger Ring, der außer dem Stickstoffatom noch weitere 0-1 Heteroatomen aus der Gruppe Sauerstoff, Stickstoff und Schwefel enthalten kann und wobei das Ringsystem zusätzlich substituiert sein kann mit N(R24)(R25), (C₁-C₆)-Alkyl oder N(R30)CO(C₁-C₆)-Alkyl;
- R24, R25: unabhängig voneinander H, (C₁-C₈)-Alkyl, -(CR34R35)ₒ-R36, (C₁-C₄)-Alkoxy-(C₁-C₄)-alkyl, (C₃-C₈)-Alkenyl, (C₃-C₈)-Alkinyl, CO-(C₁-C₈)-Alkyl, -CO-(CH₂)ₒ -R36, oder R24 und R25 bilden zusammen mit dem Stickstoffatom, an das sie gebunden sind, einen 4 bis 8-gliedrigen monocyclischen Ring welcher außer dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, (C₁-C₆)-Alkyl, O-(C₁-C₈)-Alkyl, (C₁-C₄)-Alkoxy-(C₁-C₄)-alkyl, Hydroxy-(C₁-C₄)-Alkyl, (C₀-C₈)-Alkylen-aryl, Oxo, CO(R44), N(R48)CO(C₁-C₆)-Alkyl, N(R49)(R50) oder SO₂CH₃ ;
bevorzugt unabhängig voneinander H, (C₁-C₈)-Alkyl, (C₁-C₄)-Alkoxy-(C₁-C₄)-alkyl, CO-(C₁-C₈)-Alkyl, oder R24 und R25 bilden zusammen mit dem Stickstoffatom, an das sie gebunden sind, einen 4 bis 8-gliedrigen monocyclischen Ring welcher ausser dem Stickstoffatom 0 oder 1 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl, N(R48)CO(C₁-C₆)-Alkyl oder N(R49)(R50);
besonders bevorzugt unabhängig voneinander H, (C₁-C₈)-Alkyl, CO-(C₁-C₈)-Alkyl, oder R24 und R25 bilden zusammen mit dem Stickstoffatom, an das sie gebunden sind, einen 5 bis 7-gliedrigen monocyclischen Ring welcher ausser dem Stickstoffatom noch ein weiteres Stickstoffatom enthalten kann, wobei das heterocyclische Ringsystem zusätzlich substituiert sein kann mit (C₁-C₆)-Alkyl, N(R48)CO(C₁-C₆)-Alkyl oder N(R49)(R50);
- o: 0, 1, 2, 3, 4, 5;
bevorzugt 0, 1, 2, 3, 4;
besonders bevorzugt 0, 1, 2, 3;
- R34, R35: unabhängig voneinander H, (C₁-C₈)-Alkyl, Hydroxy-(C₁-C₄)-alkyl, OH, (C₁-C₄)-Alkoxy-(C₁-C₄)-alkyl;
bevorzugt H, (C₁-C₈)-Alkyl;
besonders bevorzugt H;
- R44, R48, R49, R50: unabhängig voneinander H, (C₁-C₆)-Alkyl;
- R49 und R50: optional zusammen mit dem Stickstoffatom, an das sie gebunden sind, einen 5-6 gliedrigen Ring, welcher ausser dem Stickstoffatom noch 0-1 weitere Heteroatome aus der Gruppe N-(C₁-C₆)-Alkyl, Sauerstoff und Schwefel beinhalten kann;
- R36: O-(C₁-C₆)-Alkyl, 5-8 gliedriger monocyclischer Ring, der 0-2 Heteroatome aus der Gruppe N, O und S enthalten kann und der 5-8 gliedrige Ring weitere Substituenten wie F, Cl, Br, Oxo, O-(C₁-C₆)-Alkyl, (C₁-C₄)-Alkoxy-(C₁-C₄)-alkyl, (C₁-C₆)-Alkyl, (C₀-C₈)-Alkylen-aryl, N(R55)(R56) enthalten kann;
bevorzugt O-(C₁-C₆)-Alkyl;
- R55, R56: unabhängig voneinander H, (C₁-C₈)-Alkyl;
- R26, R30: unabhängig voneinander H, (C₁-C₆)-Alkyl.
- A: eine Gruppe der Formel -(C(R31)(R32))ₘ -, in der ein Glied ersetzt sein kann durch ein Element aus der Gruppe O, N(R33);
bevorzugt eine Bindung oder eine Gruppe der Formel -(C(R31)(R32))ₘ -, in der ein Glied ersetzt ist, durch ein Element aus der Gruppe O, N(R33);
besonders bevorzugt eine Bindung, oder eine Gruppe der Formel - (C(R31)(R32))ₘ -, in der ein Glied ersetzt ist durch ein Element aus der Gruppe O, N(R33), und die Gruppe über das Heteroatom in 4-Position (relativ zum cyclischen Harnstoff) am Cyclohexanring der Formel I bindet;
- m: 0, 1, 2, 3;
bevorzugt 0, 1, 3;
- R31, R32, R33: unabhängig voneinander H, (C₁-C₆)-Alkyl, Aryl.

Können Reste oder Substituenten mehrfach in den Verbindungen der Formel I auftreten, wie zum Beispiel R2, so können sie alle unabhängig voneinander die angegebenen Bedeutungen haben und gleich oder verschieden sein.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Alkyl-, Alkenyl- und Alkinylreste in den Substituenten R1, R2, R3, R4, R5, R6, R7, R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44, R45, R46, R46, R47, R48, R49, R50, R51, R52, R53, R54, R55, R56, R57, R58 und R59 können sowohl geradkettig oder verzweigt sein.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst.

Unter Cycloalkyl ist im Sinne der vorliegenden Anmeldung Cycloalkyl sowie Cycloalkylalkyl- (Alkyl, das wiederum mit Cycloalkyl substituiert ist z.B. Cyclopropylmethyl) zu verstehen, wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbomanyl, Bornanyl oder Adamantanyl.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 1-Propinyl, 2-Propinyl (Propargyl), 1-Butinyl, 2-Butinyl oder 3-Butinyl.

Unter Cycloalkenyl sind im Sinne der vorliegenden Anmeldung Cycloalkenylreste sowie Cycloalkenyl-Alkylreste (Alkyl, das mit Cycloalkenyl substituiert ist) zu verstehen, die mindestens drei Kohlenstoffatome enthalten. Beispiele für Cycloalkenyl sind: Cyclopentenyl, Cyclohexenyl, Cycloheptenyl und Cyclooctenyl.
Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkinylreste gilt das gleiche für die Dreifachbindungen.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Die Bezeichnung Aryl umfasst in der vorliegenden Erfindung beispielsweise auch bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Besonders bevorzugt ist Aryl Phenyl oder Naphthyl. Unter dem Begriff "Aryl" wird somit insbesondere eine Phenyl oder Naphthylgruppe verstanden.

Unter Heteroarylresten sind Reste zu verstehen, die von monocyclischen oder bicyclischen Aromaten abgeleitet sind, die Ringheteroatome, bevorzugt N, O oder S, enthalten. Im Übrigen gilt für die Heteroarylreste das bezüglich der Arylreste Aufgeführte.

Die Verbindungen der Formel I können ein oder mehrere Asymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel L Diese isomeren Formen können, wenn auch zum Teil nicht expressis verbis beschrieben, nach bekannnten Methoden erhalten werden.

Mono-, bi- oder spiro-cyclische Ringe können gesättigt, teilweise gesättigt oder ungesättigt und auch verbrückt sein.

Unter C=C ist eine Gruppe der Formel R'C=CR" zu verstehen, worin R' und R" unabhängig voneinander H, (C₁-C₈)-Alkyl, bevorzugt H, bedeuten.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Trifluormethansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z. B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionelle Derivate wie hierin beschrieben.

Können Reste oder Substituenten mehrfach in den Verbindungen der Formel I auftreten, so können sie alle unabhängig voneinander die angegebenen Bedeutungen haben und gleich oder verschieden sein.

In einer weiteren bevorzugten Ausführungsform weisen die Reste R1, R2, der Index n und die Gruppen K, E, X, D, G, A und Q die folgenden Bedeutungen auf:
- R1: (C₁-C₈)-Alkyl, (C₁-C₄)-Alkoxy-(C₁-C₄)-alkyl, ein 3 bis 10-gliedriger mono-, bi- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, (C₀-C₂)-Alkylen-aryl, Oxo, CO(R3), CON(R4)(R5), Hydroxy, N(R7)CO(C₁-C₆)-Alkyl, N(R8)(R9) oder SO₂CH₃; bevorzugt (C₁-C₈)-Alkyl, (C₁-C₄)-Alkoxy-(C₁-C₄)-alkyl, ein 3 bis 10-gliedriger mono-oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, Oxo, CO(R3), CON(R4)(R5), N(R7)CO(C₁-C₆)-Alkyl, oder SO₂CH₃;
- R3, R4, R5, R7, R8, R9: unabhängig voneinander H, (C₁-C₈)-Alkyl;
- K: eine Bindung, OCH₂, CH₂O, (C(R10)(R11))_{z}, C≡C;
- z: 1, 2, 3, 4; bevorzugt 1, 2, 3; besonders bevorzugt 1,2;
- R10, R11: unabhängig voneinander H, (C₁-C₈)-Alkyl;
- E: 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, N(R12)(R13), SO₂-CH₃, N(R16)CO(R17), N(R18)SO₂(R19), CO(R20) tragen und mono- oder bicyclisch sein kann;
bevorzugt 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, NO₂, CN, OCF₃, O-(C₁-C₆)-Alkyl, S-(C₁-C₆)-Alkyl, (C₁-C₆)-Alkyl, (C₂-C₆)-Alkenyl, O-(C₀-C₈)-Alkylen-Aryl, S-Aryl, N(R12)(R13), SO₂-CH₃, N(R16)CO(R17), CO(R20) tragen und mono- oder bicyclisch sein kann;
besonders bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R12)(R13), SO₂-CH₃, CO(R20) tragen kann
z.B. ist E ausgewählt aus der Gruppe bestehend aus 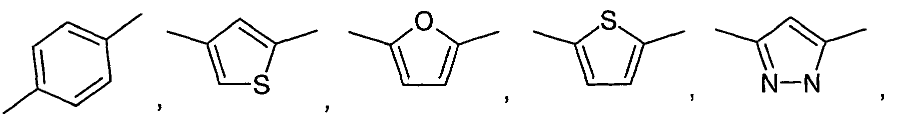 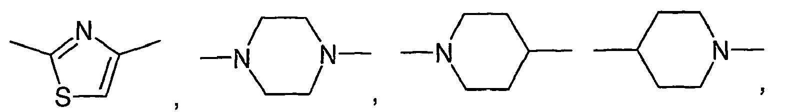 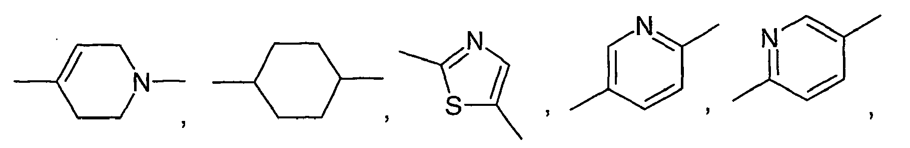 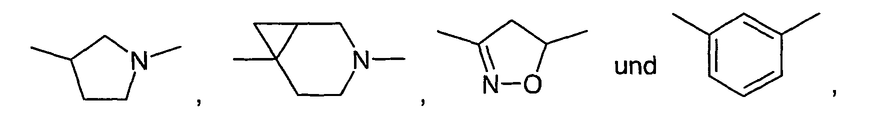
die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R12)(R13), SO₂-CH₃, CO(R20) bevorzugt H, F, Cl, Br, O-(C₁-C₆)-Alkyl, (C₁-C₆)-Alkyl, CO(R20) tragen können;
bevorzugt 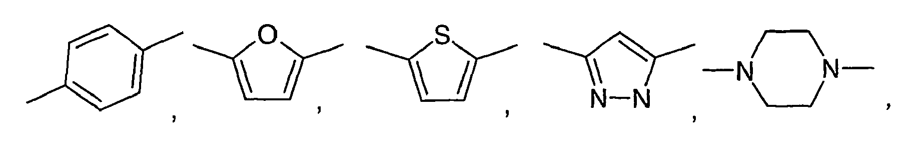 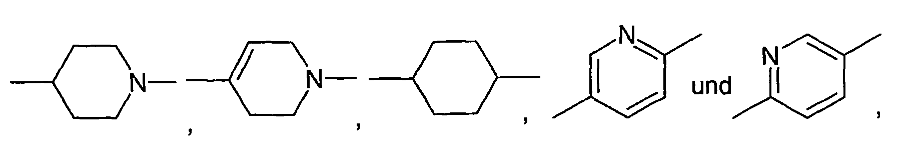 die optional die vorstehend genannten Substituenten tragen können;
besonders bevorzugt 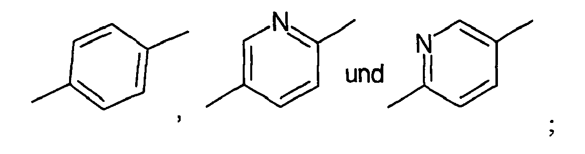
- R12, R13, R16, R18: unabhängig voneinander H, (C₁-C₈)-Alkyl ;
- R17, R19, R20: unabhängig voneinander H, (C₁-C₈)-Alkyl, Aryl; bevorzugt unabhängig voneinander H, (C₁-C₈)-Alkyl;
- X: eine Bindung, -CH₂-CH₂-; bevorzugt eine Bindung;
- D, G: entweder D ist N und G ist CH oder D ist CH und G ist N oder D und G sind gleichzeitig CH; bevorzugt sind D und G gleichzeitig CH;
- R2: OH, O-(C₁-C₆)-alkyl, O-(C₁-C₄)-Alkoxy-(C₁-C₄)-alkyl, (C₁-C₆)-Alkyl; bevorzugt O-(C₁-C₆)-Alkyl, (C₁-C₆)-Alkyl;
- n: 0, 1, 2; bevorzugt 0 oder 1;
- Q: N(R24)(R25), einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit N(R24)(R25), F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃, bevorzugt N(R24)(R25), F, Cl, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), N(R30)CO(C₁-C₆)-Alkyl oder SO₂CH₃;
- R26, R27, R28, R29, R30: unabhängig voneinander H, (C₁-C₆)-Alkyl; bevorzugt (C₁-C₆)-Alkyl;
- A: eine Gruppe der Formel -(C(R31)(R32))ₘ-, in der 0-2 Glieder ersetzt sein können durch ein Element aus der Gruppe O, N(R33), CO; bevorzugt eine Gruppe der Formel -(C(R31)(R32))ₘ-, in der 1 Glied ersetzt sein kann durch ein Element aus der Gruppe O, N(R33);
- m: 0, 1, 2, 3, 4; bevorzugt 0, 1, 3 oder 4;
- R31, R32, R33: unabhängig voneinander H, (C₁-C₆)-Alkyl, Aryl; bevorzugt H;
- R24, R25: unabhängig voneinander H, (C₁-C₈)-Alkyl, -(CH₂)ₒ-R36, CO-(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50), bevorzugt F, (C₁-C₆)-Alkyl, O-(C₁-C₈)-Alkyl, Oxo, CO(R44), N(R48)CO(C₁-C₆)-Alkyl oder N(R49)(R50);
- o: 0, 1, 2, 3, 4;
- R36: OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆)-Alkyl, O-(C₀-C₂)-Alkylen-aryl, (C₀-C₂)-Albylen-aryl und N(R55)(R56) enthalten kann; bevorzugt ein 5-8 gliedriger monocyclischer Ring, der 0-2 Heteroatome aus der Gruppe N, O und S enthalten kann und der 5-8 gliedrige Ring weitere Substituenten wie F, Oxo, O-(C₁-C₆)-Alkyl, (C₁-C₆)-Alkyl, (C₀-C₂)-Alkylen-aryl und N(R55)(R56) enthalten kann;
- R44, R45, R46, R48, R49, R50: unabhängig voneinander H, (C₁-C₆)- Alkyl;
- R55, R56: unabhängig voneinander H, (C₁-C₈)-Alkyl.

Ganz besonders bevorzugt sind D und G in den vorstehend genannten Verbindungen der Formel I jeweils CH.

In einer weiteren bevorzugten Ausführungsform betrifft die vorliegende Erfindung Verbindungen der Formel I, worin die Substituenten der Cyclohexylengruppe in paraStellung zueinander stehen, d.h., die Verbindungen der Formel I weisen die folgende Struktur Ia auf: 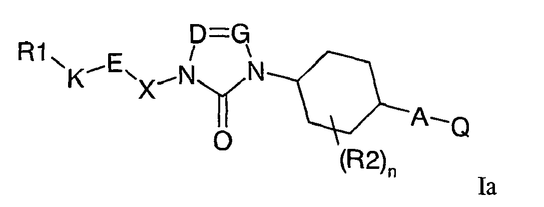 wobei die Reste R1 und R2, der Index n und die Gruppen K, E, X, D, G, A und Q die vorstehend genannten Bedeutungen aufweisen.

Des Weiteren sind Verbindungen der Formel I ganz besonders bevorzugt, worin die Gruppe Q mindestens ein Stickstoffatom enthält, wobei das Stickstoffatom in dem Ring Q enthalten sein kann und/oder in einem Substituenten des Rings Q.

Insbesondere bevorzugt sind Verbindungen der Formel I, worin die Gruppierung A-Q eine der folgenden Bedeutungen aufweist:
wenn:
   - A: eine Gruppe der Formel -(C(R31)(R32))ₘ-, in der 0-2 Glieder ersetzt sein können durch ein Element aus der Gruppe O, N(R33), CO; bevorzugt eine Gruppe der Formel -(C(R31)(R32))ₘ-, in der 1 Glied ersetzt sein kann durch ein Element aus der Gruppe O, N(R33), bedeutet;
wobei
- m: 1, 2, 3, 4; bevorzugt 1, 3 oder 4 ist;
und
- R31, R32, R33: unabhängig voneinander H, (C₁-C₆)-Alkyl, Aryl; bevorzugt H sind;
bedeutet
- Q: N(R24)(R25);
wobei
- R24, R25: unabhängig voneinander H, (C₁-C₈)-Alkyl, -(CH₂)ₒ-R36, CO-(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50), bevorzugt F, (C₁-C₆)-Alkyl, O-(C₁-C₈)-Alkyl, Oxo, CO(R44), N(R48)CO(C₁-C₆)-Alkyl oder N(R49)(R50), bedeuten;
- o: 0, 1, 2, 3, 4 ist;
- R36: OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆)-Alkyl, O-(C₀-C₂)-Alkylen-aryl, (C₀-C₂)-Alkylen-aryl und N(R55)(R56) enthalten kann, bevorzugt ein 5-8 gliedriger monocyclischer Ring, der 0-2 Heteroatome aus der Gruppe N, O und S enthalten kann und der 5-8 gliedrige Ring weitere Substituenten wie F, Oxo, O-(C₁-C₆)-Alkyl, (C₁-C₆)-Alkyl, (C₀-C₂)-Alkylen-aryl und N(R55)(R56) enthalten kann, bedeutet;
- R44, R45, R46, R48, R49, R50: unabhängig voneinander H, (C₁-C₆)-Alkyl sind;
und
- R55, R56: unabhängig voneinander H, (C₁-C₈)-Alkyl sind;
wenn:
- A: eine Gruppe der Formel -(C(R31)(R32))ₘ-, in der 0-2 Glieder ersetzt sein können durch ein Element aus der Gruppe O, N(R33), CO; bevorzugt eine Gruppe der Formel -(C(R31)(R32))ₘ-, in der 1 Glied ersetzt sein kann durch ein Element aus der Gruppe O, N(R33), bedeutet;
wobei
- m: 0, 1, 2, 3, 4; bevorzugt 0 oder 1 ist;
und
- R31, R32, R33: unabhängig voneinander H, (C₁-C₆)-Alkyl, Aryl; bevorzugt H sind;
wobei A ganz besonders bevorzugt eine Bindung oder N(R33) bedeutet;
bedeutet
- Q: einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, N0₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃; bevorzugt einen 5 bis 7-gliedrigen monocyclischen Ring, welcher ein oder zwei Stickstoffatome enthält, wobei das Ringsystem zusätzlich substituiert sein kann mit (C₁-C₆)-Alkyl, (C₁-C₄)-Akoxy-(C₁-C₄)-alkyl oder (C₀-C₂)-Alkylen-aryl;
wobei
- R26, R27, R28, R29, R30: unabhängig voneinander H, (C₁-C₆)-Alkyl; bevorzugt (C₁-C₆)-Alkyl bedeuten;
wenn:
- A: eine Gruppe der Formel -(C(R31)(R32))ₘ-, in der 0-2 Glieder ersetzt sein können durch ein Element aus der Gruppe O, N(R33), CO; bevorzugt eine Gruppe der Formel -(C(R31)(R32))ₘ-, in der 1 Glied ersetzt sein kann durch ein Element aus der Gruppe O, N(R33), bedeutet;
wobei
- m: 0, 1, 2, 3, 4; bevorzugt 0 oder 1 ist;
und
- R31, R32, R33: unabhängig voneinander H, (C₁-C₆)-Alkyl, Aryl; bevorzugt H sind;
wobei A ganz besonders bevorzugt eine Bindung oder N(R33) bedeutet;
bedeutet
- Q: einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert ist mit N(R24)(R25) und zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃; bevorzugt einen 3 bis 8-gliedrigen monocyclischen Ring welcher 0 bis 1 Heteroatom beinhalten kann, ausgewählt aus der Gruppe Sauerstoff, Stickstoff und Schwefel, wobei das Ringsystem zusätzlich substituiert ist mit N(R24)(R25) und zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃, bevorzugt F, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), N(R30)CO(C₁-C₆)-Alkyl oder SO₂CH₃;
wobei
- R26, R27, R28, R29, R30: unabhängig voneinander H, (C₁-C₆)-Alkyl; bevorzugt (C₁-C₆)-Alkyl bdeuten ;
und
- R24, R25: unabhängig voneinander H, (C₁-C₈)-Alkyl, -(CH₂)ₒ-R36, CO-(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50), bevorzugt F, (C₁-C₆)-Alkyl, O-(C₁-C₈)-Alkyl, Oxo, CO(R44), N(R48)CO(C₁-C₆)-Alkyl oder N(R49)(R50) bedeuten;
- o: 0, 1, 2, 3, 4 ist;
- R36: OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆) Alkyl, O-(C₀-C₂)-Alkylen-aryl, (C₀-C₂)-Alkylen-aryl und N(R55)(R56) enthalten kann, bevorzugt ein 5-8 gliedriger monocyclischer Ring, der 0-2 Heteroatome aus der Gruppe N, O und S enthalten kann und der 5-8 gliedrige Ring weitere Substituenten wie F, Oxo, O-(C₁-C₆)-Alkyl, (C₁-C₆)-Alkyl, (C₀-C₂)-Alkylen-aryl und N(R55)(R56) enthalten kann, bedeutet;
- R44, R45, R46, R48, R49, R50: unabhängig voneinander H, (C₁-C₆)-Alkyl sind;
und
- R55, R56: unabhängig voneinander H, (C₁-C₈)-Alkyl sind.

Im Folgenden sind beispielhaft besonders bevorzugte Gruppierungen A-Q aufgeführt: 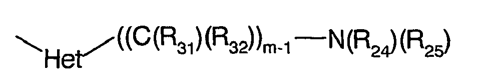 worin Het O, S, N(R33), bevorzugt 0, N(R33), und m 3 oder 4 bedeute 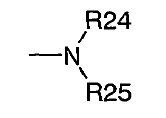 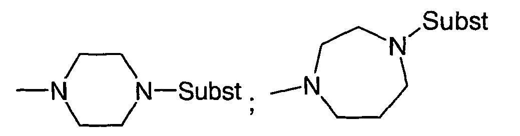 worin Subst H, (C₁-C₆)-Alkyl, Hydroxy-(C₁-C₄)-alkyl, bevorzugt H, (C₁-C₆)-Alkyl bedeutet 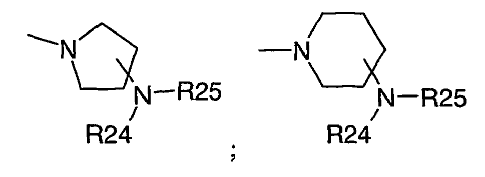 worin die Reste R24, R25, R31, R32 und R33 die vorstehend genannten Bedeutungen aufweisen.

In einer weiteren besonders bevorzugten Ausführungsform betrifft die vorliegende Erfindung Verbindungen der Formel Iaa 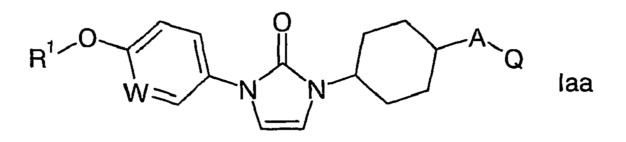 worin
- W: CH oder N bedeutet, und
der Rest R1 und die Gruppen A und Q die vorstehend genannten Bedeutungen aufweisen.

Die erfindungsgemäßen Verbindungen der Formel I können z.B. durch zu den in WO 03/057220 offenbarten Herstellungsverfahren analoge Verfahren hergestellt werden, beispielsweise durch Cyclisierungs- und/oder Kupplungsreaktionen. Geeignete Ausgangssubstanzen können nach dem Fachmann bekannten Verfahren hergestellt werden und sind zum Teil kommerziell erhältlich.

In einer bevorzugten Ausführungsform erfolgt die Herstellung der Verbindungen der Formel I durch ein Verfahren umfassend die folgenden Schritte:
a) Herstellung einer Verbindung der Formel II: der Grundkörper (1,4-Dioxaspiro[4.5]decan-8-on) ist kommerziell erhältlich. Substituierte Varianten können nach literaturbekannten Verfahren hergestellt werden (siehe z.B. Nakamura, M. et al.; J. Am. Chem. Soc. 2003, 125(21), 6362-6363; Kawafuchi, H. et al.; Tetrahedron Lett. 2002, 43(11), 2051-2054; Beauhaire, J. et al.; Tetrahedron Lett. 1995, 36(7), 1043-1046) 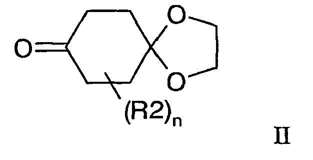
b) Umsetzung der Verbindung der Formel II mit 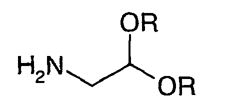 unter den Bedingungen einer reduktiven Aminierung, wobei R = niederes Alkyl, bevorzugt Ethyl oder Methyl ist, zu einer Verbindung der Formel III 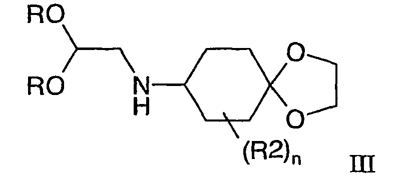
c) Kupplung der Verbindung der Formel III mit Phosgen (oder einem bekannten Phosgenäquivalent z.B. Carbonyldiimidazol) und einem primären Amin der Formel 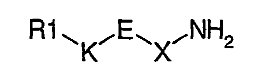 und anschließendes Entschützen der Acetale, wobei unter gleichzeitiger Cyclisierung eine Verbindung der Formel IV erhalten wird; 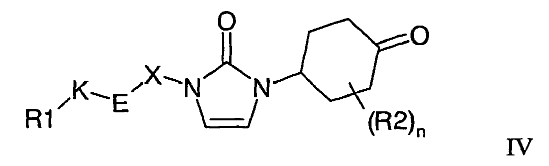
d) Umsetzung mit einem primären oder sekundären Amin unter den Bedingungen einer reduktiven Aminierung, wobei Verbindungen der Formel I erhalten werden, deren Gruppierung A-Q über ein Stickstoffatom mit dem Cyclohexylen-Ring verknüpft ist

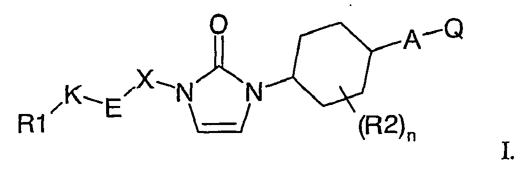

Weitere erfindungsgemäße Verbindungen der Formel I können durch analoge Verfahren erhalten werden, wobei dem Fachmann bekannt ist, wie er das vorstehend beschriebene Verfahren variieren muss, um weitere Verbindungen der Formel I zu erhalten.

Geeignete Reaktionsbedingungen und Reagenzien zur Durchführung der Schritte a) bis d) des erfindungsgemäßen Verfahrens sind dem Fachmann bekannt.

Im Folgenden sind beispielhaft zwei Wege zur Herstellung von Verbindungen der Formel I angegeben:

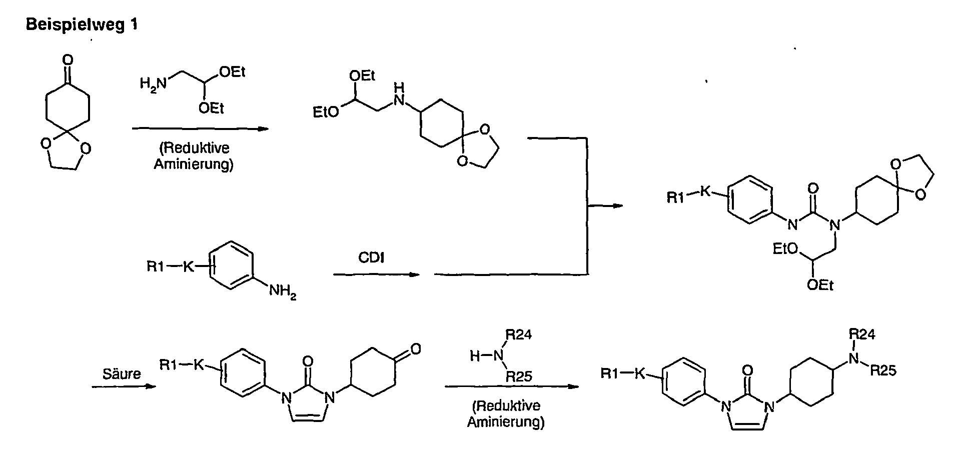

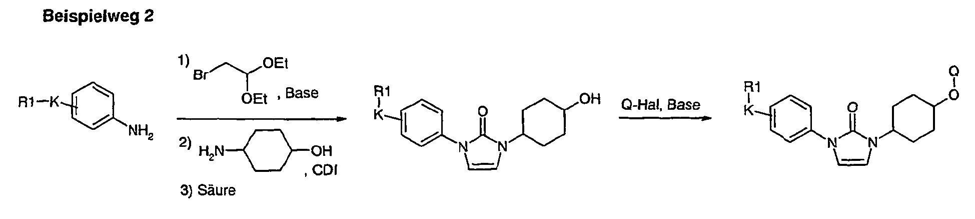

In den Beispielwegen 1 und 2 weisen R1, K und Q die vorstehend genannten Bedeutungen auf.

### Verwendung

Diese Erfindung bezieht sich weiterhin auf die Verwendung von Verbindungen der Formel I und ihren pharmazeutischen Zusammensetzungen als MCH-Rezeptor-Liganden. Die erfindungsgemäßen MCH-Rezeptor-Liganden eignen sich insbesondere als Modulatoren der Aktivität des MCH1R.

Die Rolle von MCH in der Regulation des Energiehaushalts ist inzwischen gut dokumentiert (Qu, D. et al.; Nature 1996, 380, 243-7; Shimada, M. et al. Nature 1998, 396, 670-4; Chen, Y. et al. Endocrinology 2002, 143, 2469-77; Endocrinology 2003, 144, 4831-40; Übersicht: G. Hervieu, Expert Opin. Ther. Targets 2003, 7, 495-511).

Auch gibt es Hinweise, dass MCH-Antagonisten zentral bedingte Störungen wie z.B. Depressionen günstig beeinflussen können (Borowsky, B. et al.; Nature Medicine 2002, 8, 825-30; Übersicht: G. Hervieu, Expert Opin. Ther. Targets 2003, 7, 495-511).

Besonders geeignet sind solche Verbindungen zur Behandlung und/oder Prävention von
1. Obesitas
2. Diabetes mellitus, insbesondere Typ 2 Diabetes einschließlich der Verhinderung der damit verbundenen Folgeerkrankungen. Besondere Aspekte sind dabei die
   - Hyperglykämie,
   - Verbesserung der Insulinresistenz,
   - Verbesserung der Glukose-Toleranz,
   - Schutz der β-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid- Konzentrationen
   - niedrige HDL-Cholesterin Konzentration
4. Verschiedenen anderen Zuständen, die mit dem Metabolischen Syndrom assoziert sein können, wie:
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
5. Psychiatrischen Indikationen wie
   - Depressionen
   - Angstzustände
   - Störungen des zirkadianen Rhythmus
   - Affektionsstörungen
   - Schizophrenie
   - Suchtkrankheiten

### Galenik

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg, bevorzugt 0,3 mg bis 100 mg (typischerweise von 0,01 mg bis 50 mg, bevorzugt 3 mg bis 50 mg) pro Tag pro Kilogramm Körpergewicht, z.B. 0,1 -10 mg/kg/Tag, bevorzugt 3-10 mg/kg/Tag. Eine intravenöse Dosis kann z.B. im Bereich von 0,001 mg bis 1,0 mg/kg, bevorzugt 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0,05 bis 1000 mg, typischerweise von 0,5 bis 600 mg enthalten, oder in einer weiteren Ausführungsform von 1,0 bis 1000 mg, typischerweise von 10 bis 600 mg. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird.
Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich durch ihre geringe Toxizität und ihre geringen Nebenwirkungen aus.
Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks.

### Kombinationen mit anderen Medikamenten

Bei einem weiteren Aspekt der Erfindung können die Verbindungen der Formel I in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Als weitere pharmakologisch wirksame Substanzen sind insbesondere geeignet:

### Antidiabetika

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Geeignete Antidiabetika umfassen alle Insuline und Insulinderivate, wie z.B. Lantus® oder HMR 1964 oder Apidra^{®}, sowie andere schnell wirkende Insuline (siehe z.B. US 6 221 633), Amylin, GLP-1- und GLP-2-Derivate, wie in WO 01/04146 beschrieben oder auch wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Glukagon-Rezeptor-Antagonisten, GLP-1-Agonisten, DPP-IV Inhibitoren, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren der GSK3-beta, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen, die zur Veränderung der Lipidzusammensetzung des Blutes führen, wie antihyperlipidämische Wirkstoffe und antilipidämische Wirkstoffe, z.B. HMGCoA-Reduktase-Inhibitoren. Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme und/oder Nahrungsmittelaufnahme verringern, PPAR- und RXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I verabreicht in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat).

Bei einer Ausführungsform der Erfindung werden die Verbindungen der Formel I in Kombination mit einem PPAR gamma Agonisten, wie z.B. Rosiglitazon oder Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin oder Rosuvastatin verabreicht.

Bei einer Ausführungsform der Erfindung werden die Verbindungen der Formel I in Kombination mit einem Gallensäureresorptionsinhibitor verabreicht (siehe z.B. US 6 245 744, US 6 221 897, US 6 277 831, EP 0 683 773, EP 0 683 774).

Bei einer Ausführungsform der Erfindung werden die Verbindungen der Formel I in Kombination mit einem polymeren Gallensäureadsorber, wie z.B. Cholestyramin oder Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6 342 512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPAR alpha Agonisten verabreicht.

Bei einer Ausführungsform der Erfindung werden die Verbindungen der Formel I in Kombination mit einem Fibrat, wie z.B. Fenofibrat, Gemfibrozil, Clofibrat oder Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonisten verabreicht.

### Autiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin.

Bei noch einer Ausführungsform ist der weitere Wirkstoff Sibutramin.
Bei einer anderen Ausführungsform ist der weitere Wirkstoff Rimonabant.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo- 2,3,3a,4,6,7-hexahydro-pyrazolo[4,3-c]pyridin-5-yl)-1-(4-chloro-phenyl)-2-oxo-ethyl]- amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), CB1-Antagonisten /Inversen Agonisten, H3-Antagonisten / Inversen Agonisten (z.B. 3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethylphenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)) ; Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), BRS3-Agonisten, Galanin-Antagonisten, Ghrelin-Antagonisten, MCH-Antagonisten, mGluR5-Antagonisten, Opioid-Antagonisten, Wachstumshormon (z.B. humanes Wachstumshormon), Wachstumshormon freisetzende Verbindungen (6-Benzyloxy-l-(2-diisopropylamino-ethylcarbamoyl)-3,4-dihydro-1H-isoquinoline-2-carboxylic acid tert-butyl ester (WO 01/85695)), CNTF, CNTF-Derivaten (z.B. Axokine), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (z.B. Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

In zwei gleichzeitig erschienenen Artikeln in Nature (Nature 400, 261-264, 1999; Nature 400, 265-269, 1999) wurde separat von zwei Arbeitsgruppen ein hochspezifischer Rezeptor für das Melanin-Concentrating-Hormone (MCH) beschrieben. MCH übernimmt wichtige Funktionen bei der Steuerung der Nahrungsaufnahme. Verbindungen, die auf den MCH-Rezeptor wirken, besitzen daher eine anorektische Wirkung und sind zur Behandlung von Obesitas geeignet. Die Prüfung auf anorektische Wirkung der erfindungsgemäßen Verbindungen der Formel I wurde daher wie folgt durchgeführt.

### Funktionelle Messungen zur Ermittlung von IC50-Werten

Die Klonierung der cDNA für den humanen MCH-Rezeptor, Herstellung einer rekombinanten HEK293-Zellinie, welche den humanen MCH-Rezeptor exprimiert, sowie funktionelle Messungen mit der rekombinanten Zellinie erfolgten sinngemäß wie von Audinot et al. (J. Biol. Chem. 276 13554-13562, 2001) beschrieben. Im Unterschied zur Literaturstelle wurde jedoch für die Konstruktion des Expressionsvektors das Plasmid pEAK8 der Fa. EDGE Biosystems (USA) verwendet. Als Wirt für die Transfektion diente eine transformierte HEK-Zellinie namens "PEAK Stable Cells" (ebenfalls von EDGE Biosystems). Die funktionellen Messungen des zellulären Calciumflusses nach Agonistenzugabe (MCH) in Gegenwart von erfindungsgemäßem Ligand erfolgten mit Hilfe des FLIPR-Gerätes der Fa. Molecular Devices (USA), unter Verwendung von Vorschriften des Geräteherstellers.

### Prüfung auf biologische Aktivität

Für die Beispielverbindungen 1, 2, 5 und 8 wurden unter den vorstehend genannten Bedingungen IC50-Werte in der Größenordnung von 0,01 bis 2 *µ*M gemessen. Die Beispielverbindungen 3, 6 und 9 zeigten IC50-Werte von 2 bis 10 *µ*M.

Die nachfolgend aufgeführten Beispiele und Herstellungsmethoden dienen zur Erläuterung der Erfindung, ohne diese jedoch einzuschränken.

### Beispiele

### Allgemeine Erläuterungen

### a) Zeichenweise der Strukturformeln

In den Strukturformeln der gegebenen Beispiele sind zur Übersichtlichkeit nur Nicht-Wasserstoffatome dargestellt.

### b) Salzformen

Viele der erfindungsgemäßen Verbindungen sind Basen und können mit entsprechend starken Säuren Salze bilden. Insbesondere können die Verbindungen nach HPLCchromatographischer Reinigung unter Verwendung eines Trifluoressigsäure enthaltenden Laufmittels als Hydrotrifluoracetate vorliegen. Diese können durch einfaches Behandeln einer Lösung der Salze z. B. mit Natriumcarbonatlösung in die gezeigten freien Basen überführt werden.

### c) Einheiten der Charakterisierungsdaten

Die Einheit der angegebenen Molekulargewichte ist "g/mol". Beobachtete Peaks im Massenspektrum sind angegeben als ganzzahliger Quotient der molaren Molekülionmasse und der Ladung des Molekülions (m/z).

### Abkürzungen

Wenn nicht anders angegeben, haben die Abkürzungen in den nachstehenden Beispielen folgende Bedeutung:
NaBH₃CN = Natriumcyanoborhydrid
DMF = N,N-Dimethylformamid
EDC =1-(3-Dimethylaminopropyl)-3-ethylcarbodiimide
THF = Tetrahydrofuran
DMSO = Dimethylsulfoxid
HOBt = 1-Hydroxybenzotriazol
HOAt = 1-Hydroxy-7-azabenzotriazol
HCl = Salzsäure
HPLC = Hochleistungs-Flüssigkeitschromatographie
PyBOP = Benzotriazol-1-yloxytris(pyrrolidino)phosphonium hexafluorophospat
CDI = Carbonyldiimidazol

### Beispiel 1

### (R)-N-(1-{4-[2-Oxo-3-(4-phenoxy-phenyl)-2,3-dihydro-imidazol-1-yl]-cyclohexyl}-pyrrolidin-3- yl)-acetamid

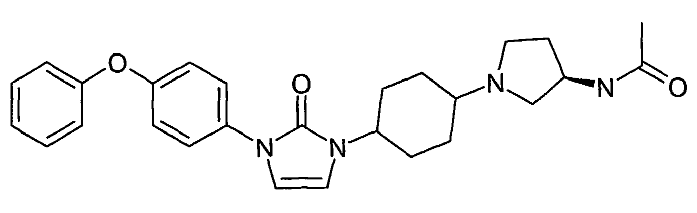

### Methode A

Eine Mischung von 1-(4-Oxo-cyclohexyl)-3-(4-phenoxy-phenyl)-1,3-dihydro-imidazol-2-on (348 mg), (R)-N-Pyrrolidin-3-yl-acetamid (128 mg) und Dichlorethan (5 mL) wurde mit Natrium-triacetoxyborhydrid (295 mg) versetzt und für 48 Stunden gerührt. Es wurde Natronlauge (1M) zugesetzt und die Mischung mit Ethylacetat extrahiert. Die organische Phase wurde über Magnesiumsulfat getrocknet, filtriert und eingeengt. Der Rückstand wurde durch präparative HPLC gereinigt. Man erhielt so das Produkt mit dem Molekulargewicht 460,58 (C₂₇H₃₂N₄O₃); MS (ESI): 461 ([M+H]⁺).

### 1-(4-Oxo-cyclohexyl)-3-(4-phenoxy-phenyl)-1,3-dihydro-imidazol-2-on

### Methode B

Eine Lösung von Carbonyldiimidazol (1,8 g) in DMF (30 mL) bei 0°C wurde mit einer Lösung von 4-Phenoxyanilin (2,0 g) in DMF (10 mL) versetzt. Nach 30 Minuten wurde (2,2-Diethoxy-ethyl)-(1,4-dioxa-spiro[4.5]dec-8-yl)-amin (3,0 g) in DMF (10 mL) zugesetzt und die Mischung für 30 Minuten auf 80°C erwärmt. Es wurde Trifluoressigsäure (5 mL) zugesetzt und die Mischung für weitere 5 Stunden bei 80°C gehalten. Nach dem Abkühlen wurde die Mischung mit Wasser verdünnt und mit Ethylacetat extrahiert. Die organische Phase wurde über Magnesiumsulfat getrocknet, filtriert und eingeengt. Man erhielt so das Produkt mit dem Molekulargewicht 348,41 (C₂₁H₂₀N₂O₃) ; MS (ESI) : 349 ([M+H]⁺).
Analog wurden 1-(4-Cyclopentyloxy-phenyl)-3-(4-oxo-cyclohexyl)-1,3-dihydro-imidazol-2-on aus 4-Cyclopentyloxy-anilin, 1-(6-Cyclopentyloxy-pyridin-3-yl)-3-(4-oxo-cyclohexyl)-1,3-dihydro-imidazol-2-on aus 6-Cyclopentyloxy-pyridin-3-ylamin und 1-(4-Butoxy-phenyl)-3-(4-oxo-cyclohexyl)-1,3-dihydro-imidazol-2-on aus 4-Butoxy-anilin erhalten.

### (2,2-Diethoxy-ethyl)-(1,4-dioxa-spiro[4.5]dec-8-yl)-amin

Nach Methode A wurde 1,4-Dioxa-spiro[4.5]decan-8-on (1,0 g) mit 2,2-Diethoxyethylamin (0,85 g) umgesetzt. Man erhielt so das Produkt mit dem Molekulargewicht 273,38 (C₁₄H₂₇NO₄); MS (ESI): 274 ([M+H]⁺).

### 6-Cyclopentyloxy-pyridin-3-ylamin

Eine Mischung aus 5-Nitro-pyridin-2-ol (14,0 g), Bromcyclopentan (8,0 g), Kaliumcarbonat (14 g) und DMF (200 mL) wurde für 6 Stunden auf 80 °C erwärmt. Nach dem Abkühlen wurde die Reaktionsmischung mit Wasser verdünnt und mit Ethylacetat extrahiert. Die organische Phase wurde mit Wasser gewaschen, über Magnesiumsulfat getrocknet und eingeengt. Der Rückstand wurde durch Chromatographie an Kieselgel gereinigt. Das erhaltene Produkt (2-Cyclopentyloxy-5-nitro-pyridin) wurde in Ethanol unter Verwendung von Palladium(II)-hydroxid als Katalysator hydriert. Man erhielt so das Produkt mit dem Molekulargewicht 178,24 (C10H14N2O); MS (ESI): 179 (M+H+).

### 4-Cyclopentyloxy-anilin

Eine Mischung von 4-Nitrophenol (63,7 g), Bromcyclopentan (68,2 g), Kaliumcarbonat (63,3 g) und DMF (300 mL) wurde für 24 Stunden auf 80 °C erwärmt. Nach dem Abkühlen wurde mit Wasser verdünnt und mit Ethylacetat extrahiert. Die organische Phase wurde mit Wasser gewaschen, über Magnesiumsulfat getrocknet und eingeengt. Der Rückstand wurde in Ethanol unter Verwendung von Palladium(II)-hydroxid als Katalysator hydriert. Man erhielt so das Produkt mit dem Molekulargewicht 177,25 (C11H15NO) ; MS (ESI): 178 (M+H+).

### Beispiel 2

### Trans-1-(4-Cyclopentyloxy-phenyl)-3-[4-(2-dimethylamino-ethoxy)-cyclohexyl]-1,3-dihydro- imidazol-2-one

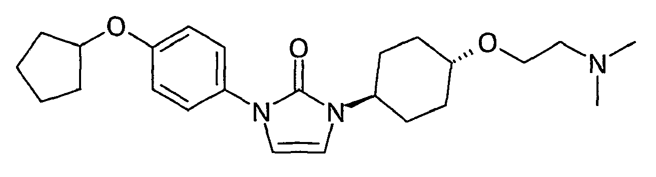

Eine Mischung von trans-1-(4-Cyclopentyloxy-phenyl)-3-(4-hydroxy-cyclohexyl)-1,3-dihydro-imidazol-2-one (0,30 g), und DMF (10 mL) wurde mit Natriumhydrid (42 mg) versetzt und nach beendeter Gasentwicklung Dimethylaminoethylchlorid (94 mg) zugesetzt. Nach acht Stunden wurde die Reaktionslösung mit Wasser verdünnt und die Mischung mit Ethylacetat extrahiert. Die organische Phase wurde über Magnesiumsulfat getrocknet, filtriert und eingeengt. Der Rückstand wurde durch präparative HPLC gereinigt. Man erhielt so das Produkt mit dem Molekulargewicht 413,56 (C₂₄H₃₅N₃O₃); MS (ESI): 414 ([M+H]⁺).

### Trans-1-(4-Cyclopentyloxy-phenyl)-3-(4-hydroxy-cyclohexyl)-1,3-dihydro-imidazol-2-one

Nach Methode B wurde trans-4-Aminocyclohexanol mit CDI und (4-Cyclopentyloxy-phenyl)-(2,2-diethoxy-ethyl)-amine umgesetzt. Das isolierte Rohprodukt wurde mit DMF (2 mL) und TFA (2 mL) versetzt und für 12 Stunden stehen gelassen. Die Reaktionslösung wurde mit Wasser verdünnt und die Mischung mit Ethylacetat extrahiert. Die organische Phase wurde über Magnesiumsulfat getrocknet, filtriert und eingeengt. Der Rückstand wurde durch präparative HPLC gereinigt. Man erhielt so das Produkt mit dem Molekulargewicht 342,44 (C₂₀H₂₆N₂O₃); MS (ESI): 343 ([M+H]⁺).

### (4-Cyclopentyloxy-phenyl)-(2,2-diethoxy-ethyl)-amine

Eine Suspension von 4-Cyclopentyloxy-anilin (8,86 g), Bromacetaldehyddiethylacetal (13 g), Kaliumcarbonat (13,8 g) und Dimethylformamid (100 mL) wurde für 6 Stunden auf 80 °C erhitzt. Nach dem Abkühlen wurde filtriert und das Filtrat eingeengt. Der Rückstand wurde durch Chromatographie an Kieselgel (Eluent: Heptan/Ethylacetat 2:1) gereinigt. Man erhielt so das Produkt mit dem Molekulargewicht 293,41 (C₁₇H₂₇NO₃) ; MS (ESI): 294 ([M+H]⁺).

Nach Methode A wurden unter Verwendung der entsprechenden Ketone und Amine die folgenden Verbindungen hergestellt:

| Bsp. No. | Struktur | Summenformel | Molekulargewicht | ESI-MS [M+H]⁺ |
|---|---|---|---|---|
| 3 | 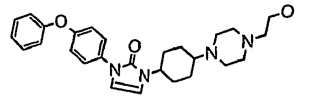 | C27H34N4O3 | 462,60 | 463 |
| 4 | 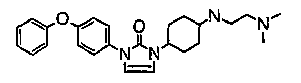 | C25H32N4O2 | 420,56 | 421 |
| 5 | 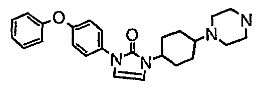 | C25H30N4O2 | 418,54 | 419 |
| 6 | 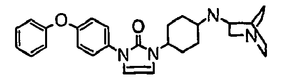 | C28H34N4O2 | 458,61 | 459 |
| 7 | 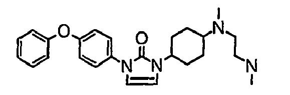 | C25H32N4O2 | 420,56 | 421 |
| 8 | 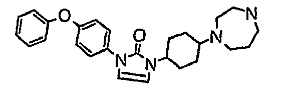 | C26H32N4O2 | 432,57 | 433 |
| 9 | 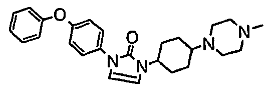 | C26H32N4O2 | 432,57 | 433 |
| 10 | 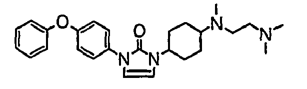 | C26H34N4O2 | 434,59 | 435 |
| 11 | 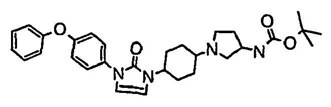 | C30H38N4O4 | 518,66 | 519 |
| 12 | 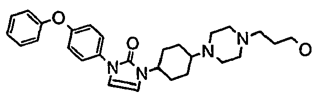 | C28H36N4O3 | 476,62 | 477 |
| 13 | 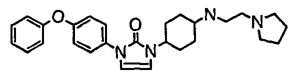 | C27H34N4O2 | 446,60 | 447 |
| 14 | 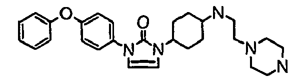 | C27H35N5O2 | 461,61 | 462 |
| 15 | 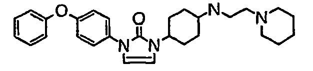 | C28H36N4O2 | 460,62 | 461 |
| 16 | 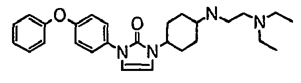 | C27H36N4O2 | 448,61 | 449 |
| 17 | 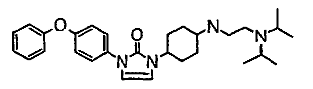 | C29H40N4O2 | 476,67 | 477 |
| 18 | 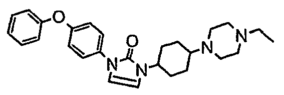 | C27H34N4O2 | 446,60 | 447 |
| 19 | 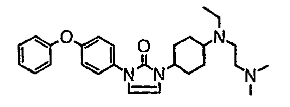 | C27H36N4O2 | 448,61 | 449 |
| 20 | 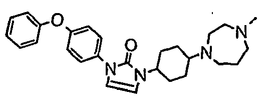 | C27H34N4O2 | 446,60 | 447 |
| 21 | 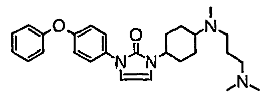 | C27H36N4O2 | 448,61 | 449 |
| 22 | 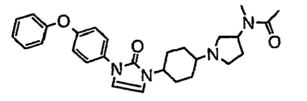 | C28H34N4O3 | 474,61 | 475 |
| 23 | 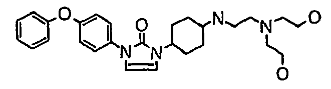 | C27H36N4O4 | 480,61 | 481 |
| 24 | 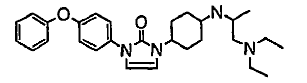 | C28H38N4O2 | 462,64 | 463 |
| 25 | 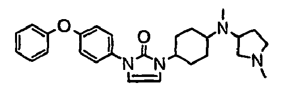 | C27H34N4O2 | 446,60 | 447 |
| 26 | 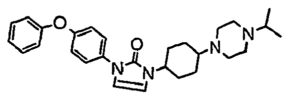 | C28H36N4O2 | 460,62 | 461 |
| 27 | 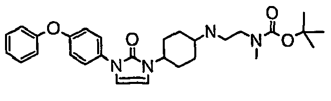 | C29H38N4O4 | 506,65 | 507 |
| 28 | 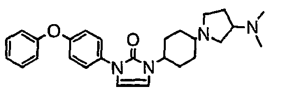 | C27H34N4O2 | 446,60 | 447 |
| 29 | 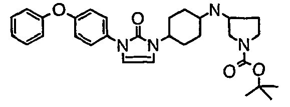 | C30H38N4O4 | 518,66 | 519 |
| 30 | 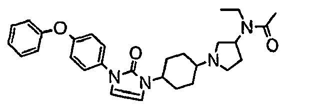 | C29H36N4O3 | 488,64 | 489 |
| 31 | 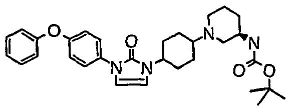 | C31H40N4O4 | 532,69 | 533 |
| 32 | 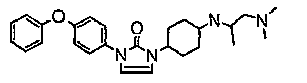 | C26H34N4O2 | 434,59 | 435 |
| 33 | 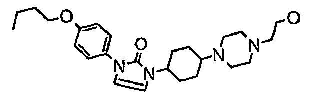 | C25H38N4O3 | 442,61 | 443 |
| 34 | 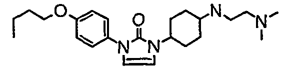 | C23H36N4O2 | 400,57 | 401 |
| 35 | 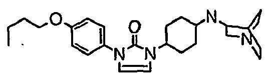 | C26H38N4O2 | 438,62 | 439 |
| 36 | 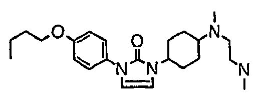 | C23H36N4O2 | 400,57 | 401 |
| 37 | 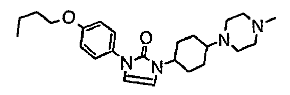 | C24H36N4O2 | 412,58 | 413 |
| 38 | 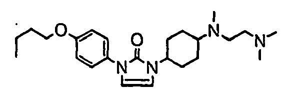 | C24H38N4O2 | 414,60 | 415 |
| 39 | 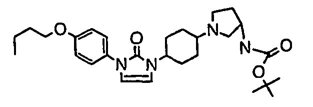 | C28H42N4O4 | 498,67 | 499 |
| 40 | 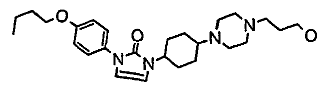 | C26H40N4O3 | 456,63 | 457 |
| 41 | 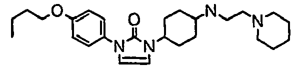 | C26H40N4O2 | 440,63 | 441 |
| 42 | 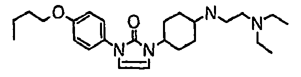 | C25H40N4O2 | 428,62 | 429 |
| 43 | 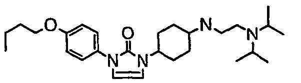 | C27H44N4O2 | 456,68 | 457 |
| 44 | 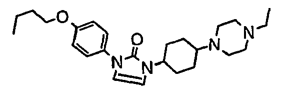 | C25H38N4O2 | 426,61 | 427 |
| 45 | 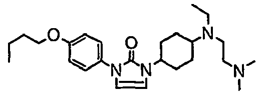 | C25H40N4O2 | 428,62 | 429 |
| 46 | 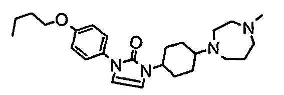 | C25H38N4O2 | 426,61 | 427 |
| 47 | 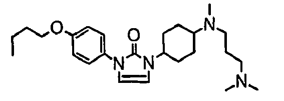 | C25H40N4O2 | 428,62 | 429 |
| 48 | 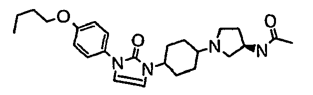 | C25H36N4O3 | 440,59 | 441 |
| 49 | 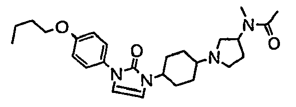 | C26H38N4O3 | 454,62 | 455 |
| 50 | 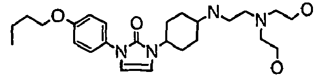 | C25H40N4O4 | 460,62 | 461 |
| 51 | 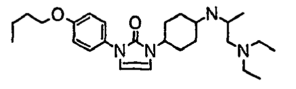 | C26H42N4O2 | 442,65 | 443 |
| 52 | 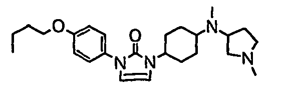 | C25H38N4O2 | 426,61 | 427 |
| 53 | 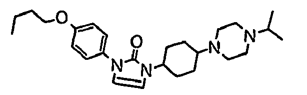 | C26H40N4O2 | 440,63 | 441 |
| 54 | 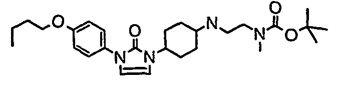 | C27H42N4O4 | 486,66 | 487 |
| 55 | 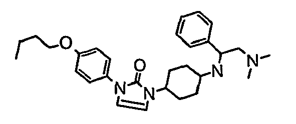 | C29H40N4O2 | 476,67 | 477 |
| 56 | 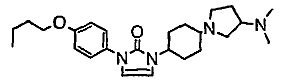 | C25H38N4O2 | 426,61 | 427 |
| 57 | 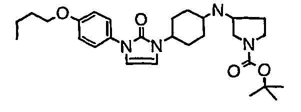 | C28H42N4O4 | 498,67 | 499 |
| 58 | 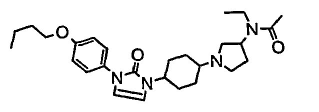 | C27H40N4O3 | 468,64 | 469 |
| 59 | 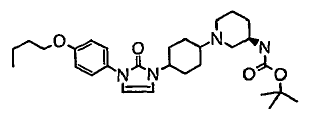 | C29H44N4O4 | 512,70 | 513 |
| 60 | 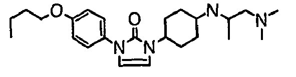 | C24H38N4O2 | 414,60 | 415 |
| 61 | 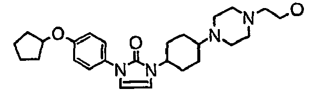 | C26H38N4O3 | 454,62 | 455 |
| 62 | 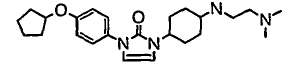 | C24H36N4O2 | 412,58 | 413 |
| 63 | 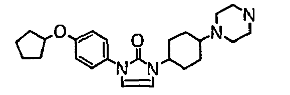 | C24H34N4O2 | 410,56 | 411 |
| 64 | 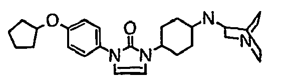 | C27H38N4O2 | 450,63 | 451 |
| 65 | 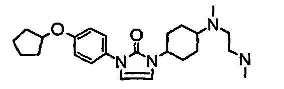 | C24H36N4O2 | 412,58 | 413 |
| 66 | 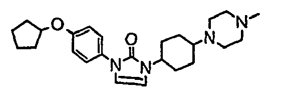 | C25H36N4O2 | 424,59 | 425 |
| 67 | 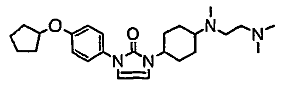 | C25H38N4O2 | 426,61 | 427 |
| 68 | 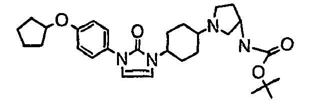 | C29H42N4O4 | 510,68 | 511 |
| 69 | 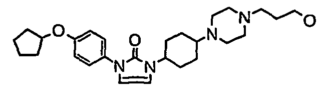 | C27H40N4O3 | 468,64 | 469 |
| 70 | 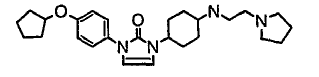 | C26H38N4O2 | 438,62 | 439 |
| 71 | 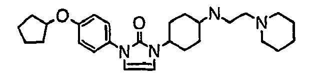 | C27H40N4O2 | 452,65 | 453 |
| 72 | 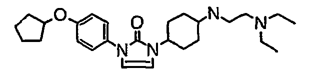 | C26H40N4O2 | 440,63 | 441 |
| 73 | 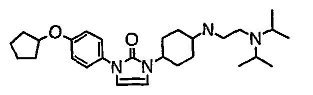 | C28H44N4O2 | 468,69 | 469 |
| 74 | 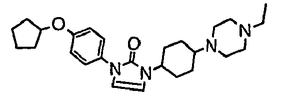 | C26H38N4O2 | 438,62 | 439 |
| 75 | 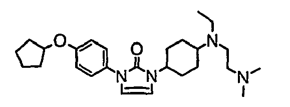 | C26H40N4O2 | 440,63 | 441 |
| 76 | 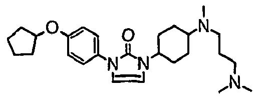 | C26H40N4O2 | 440,63 | 441 |
| 77 | 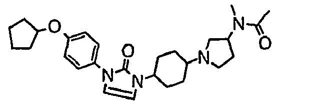 | C27H38N4O3 | 466,63 | 467 |
| 78 | 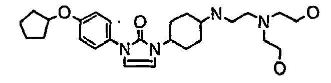 | C26H40N4O4 | 472,63 | 473 |
| 79 | 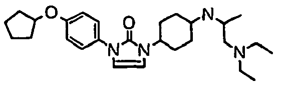 | C27H42N4O2 | 454,66 | 455 |
| 80 | 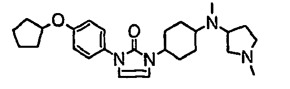 | C26H38N4O2 | 438,62 | 439 |
| 81 | 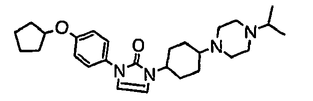 | C27H40N4O2 | 452,65 | 453 |
| 82 | 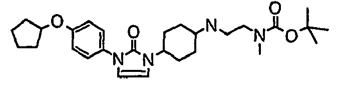 | C28H42N4O4 | 498,67 | 499 |
| 83 | 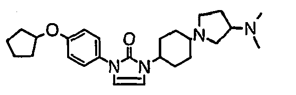 | C26H38N4O2 | 438,62 | 439 |
| 84 | 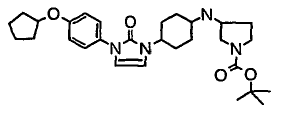 | C29H42N4O4 | 510,68 | 511 |
| 85 | 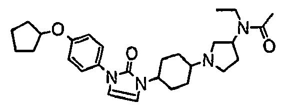 | C28H40N4O3 | 480,66 | 481 |
| 86 | 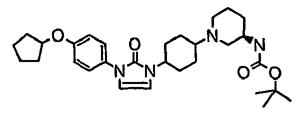 | C30H44N4O4 | 524,71 | 525 |
| 87 | 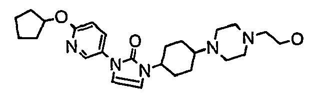 | C25H37N5O3 | 455,61 | 456 |
| 88 | 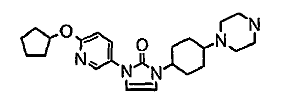 | C23H33N5O2 | 411,55 | 412 |
| 89 | 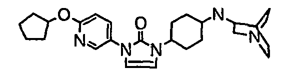 | C26H37N5O2 | 451,62 | 452 |
| 90 | 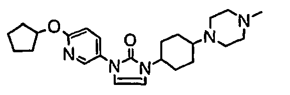 | C24H35N5O2 | 425,58 | 426 |
| 91 | 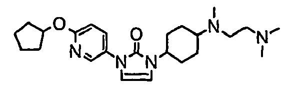 | C24H37N5O2 | 427,59 | 428 |
| 92 | 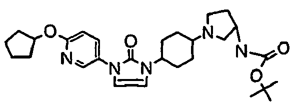 | C28H41N5O4 | 511,67 | 512 |
| 93 | 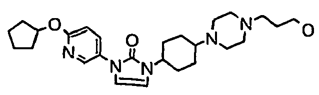 | C26H39N5O3 | 469,63 | 470 |
| 94 | 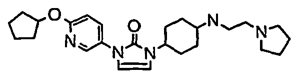 | C25H37N5O2 | 439,61 | 440 |
| 95 | 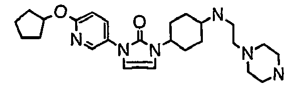 | C25H38N6O2 | 454,62 | 455 |
| 96 | 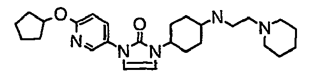 | C26H39N5O2 | 453,63 | 454 |
| 97 | 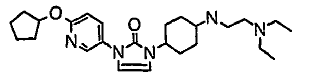 | C25H39N5O2 | 441,62 | 442 |
| 98 | 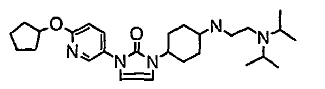 | C27H43N5O2 | 469,68 | 470 |
| 99 | 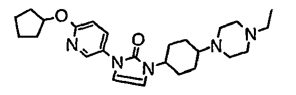 | C25H37N5O2 | 439,61 | 440 |
| 100 | 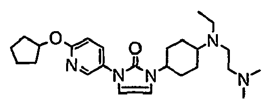 | C25H39N5O2 | 441,62 | 442 |
| 101 | 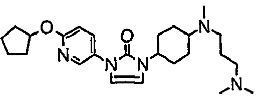 | C25H39N5O2 | 441,62 | 442 |
| 102 | 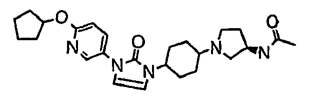 | C25H35N5O3 | 453,59 | 454 |
| 103 | 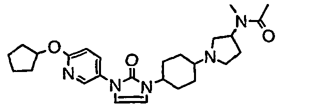 | C26H37N5O3 | 467,62 | 468 |
| 104 | 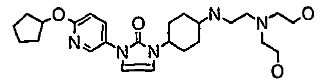 | C25H39N5O4 | 473,62 | 474 |
| 105 | 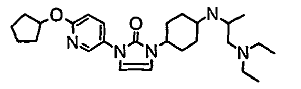 | C26H41N5O2 | 455,65 | 456 |
| 106 | 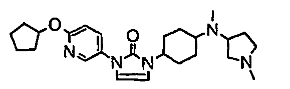 | C25H37N5O2 | 439,61 | 440 |
| 107 | 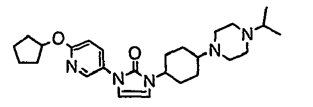 | C26H39N5O2 | 453,63 | 454 |
| 108 | 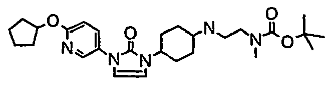 | C27H41N5O4 | 499,66 | 500 |
| 109 | 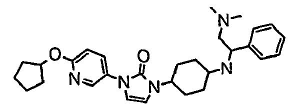 | C29H39N5O2 | 489,67 | 490 |
| 110 | 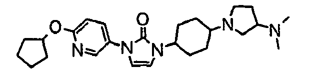 | C25H37N5O2 | 439,61 | 440 |
| 111 | 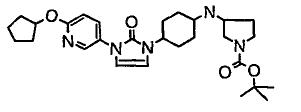 | C28H41N5O4 | 511,67 | 512 |
| 112 | 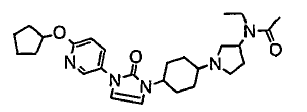 | C27H39N5O3 | 481,64 | 482 |
| 113 | 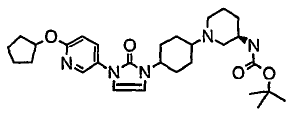 | C29H43N5O4 | 525,70 | 526 |
| 114 | 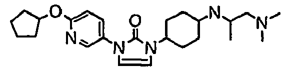 | C24H37N5O2 | 427,59 | 428 |
| 115 | 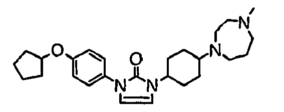 | C26H38N4O2 | 438,62 | 439 |
| 116 | 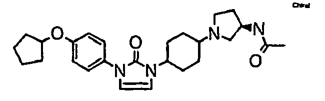 | C26H36N4O3 | 452,60 | 453 |
| 117 | 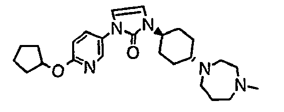 | C25H37N5O2 | 439,61 | 440 |
| 118 | 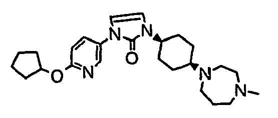 | C25H37N5O2 | 439,61 | 440 |

## Patentansprüche

1. Verbindungen der Formel I, 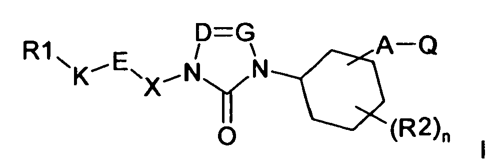 worin bedeuten
R1 H, (C₁-C₈)-Alkyl, (C₁-C₄)-Alkoxy-(C₁-C₄)-alkyl, (C₃-C₈)-Alkenyl, (C₃-C₈)-Alkinyl, einen 3 bis 10-gliedrigen mono-, bi- oder spirocyclischen Ring welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, NO₂, CN, (C₁-C₆)-Alkyl, O-(C₁-C₈)-Alkyl, S-(C₁-C₈)-Alkyl, (C₁-C₄)-Alkoxy-(C₁-C₄)-alkyl, (C₀-C₈)-Alkylen-aryl, Oxo, CO(R3), CON(R4)(R5), Hydroxy, Hydroxy-(C₁-C₄)-alkyl, COO(R6), N(R7)CO(C₁-C₆)-Alkyl, N(R8)(R9) oder SO₂CH₃;
R3, R4, R5, R6, R7, R8, R9, unabhängig voneinander H, (C₁-C₆)-Alkyl;
K eine Gruppe der Formel -(CR10R11)_{z}-, worin eine oder mehrere Gruppen -(CR10R11)- durch Z ersetzt sein können, eine Bindung, C≡C, C=C;
Z O, CO, N(R59), S, SO, SO₂;
R10, R11 unabhängig voneinander H, (C₁-C₈)-Alkyl, Hydroxy-(C₁-C₄)-Alkyl, Hydroxy, (C₁-C₄)-Alkoxy-(C₁-C₄)-Alkyl, wobei R10 und R11 in den z Gruppen jeweils die gleichen oder verschiedene Bedeutungen aufweisen können;
z 1, 2, 3, 4, 5, 6;
R59 H, (C₁-C₈)-Alkyl ;
E 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, NO₂, CN, OCF₃, Oxo, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)-Alkylen-aryl, O-(C₀-C₈)-Alkylen-aryl, S-Aryl, N(R12)(R13), SO₂-CH₃, COOH, COO-(C₁-C₆)-Alkyl, CON(R14)(R15), N(R16)CO(R17), N(R18)SO₂(R19), CO(R20) tragen und mono- oder bicyclisch sein kann;
R12, R13, R14, R15, R16, R18 unabhängig voneinander H, (C₁-C₈)-Alkyl;
oder
R12 und R13, R14 und R15 unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe N-(C₁-C₆)-Alkyl, Sauerstoff und Schwefel beinhalten kann;
R17, R19, R20 unabhängig voneinander H, (C₁-C₈)-Alkyl, Aryl;
X eine Bindung, eine Gruppe der Formel -(CR21 R22)y-, worin eine oder mehrere Gruppen -(CR21 R22)- durch Y ersetzt sein können;
Y O, CO, N(R23), S, SO, SO₂;
R21, R22 unabhängig voneinander H, (C₁-C₄)-Alkyl, wobei R21 und R22 in den y Gruppen jeweils die gleichen oder verschiedene Bedeutungen aufweisen können;
y 1, 2, 3, 4, 5, 6;
R23 H, (C₁-C₈)-Alkyl;
D, G CH oder N;
R2 OH, O-(C₁-C₆)-Alkyl, O-(C₁-C₄)-Alkoxy-(C₁-C₄)-alkyl, (C₁-C₆)-Alkyl;
n 0, 1, 2, 3, 4;
Q N(R24)(R25), einen 3 bis 8-gliedrigen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit (C₀-C₄)-Alkylen-N(R24)(R25), F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃;
R26, R27, R28, R29, R30 unabhängig voneinander H, (C₁-C₆)-Alkyl;
A eine Gruppe der Formel -(C(R31)(R32))ₘ-, in der 0-2 Glieder ersetzt sein können durch ein Element aus der Gruppe O, S, N(R33), CO, SO₂;
m 0, 1, 2, 3, 4, 5;
R31, R32, R33 unabhängig voneinander H, (C₁-C₆)-Alkyl, Aryl;
R24, R25 unabhängig voneinander H, (C₁-C₈)-Alkyl, -(CR34R35)ₒ-R36, (C₁-C₄)-Alkoxy-(C₁-C₄)-alkyl, (C₃-C₈)-Alkenyl, (C₃-C₈)-Alkinyl, CO-(C₁-C₈)-Alkyl, -CO-(CH₂)ₒ -R36, CO(C(R37)(R38))_{q}N(R39)(R40), CO(C(R41)(R42))ₛO(R43);
oder R24 und R25 bilden zusammen mit dem Stickstoffatom, an das sie gebunden sind, einen 4 bis 10-gliedrigen mono-, bi- oder spirocyclischen Ring welcher ausser dem Stickstoffatom 0 bis 4 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, N0₂, CN, (C₁-C₆)-Alkyl, O-(C₁-C₈)-Alkyl, (C₁-C₄)-Alkoxy-(C₁-C₄)-alkyl, Hydroxy-(C₁-C₄)-Alkyl, (C₀-C₈)-Alkylen-aryl, Oxo, CO(R44), CON(R45)(R46), Hydroxy, COO(R47), N(R48)CO(C₁-C₆)-Alkyl, N(R49)(R50) oder S0₂CH₃;
o 0, 1, 2, 3, 4, 5, 6;
q, s unabhängig voneinander 0, 1, 2, 3, 4;
R34, R35 unabhängig voneinander H, (C₁-C₈)-Alkyl, Hydroxy-(C₁-C₄)-Alkyl, OH, (C₁-C₄)-Alkoxy-(C₁-C₄)-alkyl ;
R37, R38, R39, R40, R41, R42, R43, R44, R45, R46, R47, R48, R49, R50 unabhängig voneinander H, (C₁-C₆)-Alkyl;
R39 und R40, R45 und R46, R49 und R50 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe N-(C₁-C₆)-Alkyl, Sauerstoff und Schwefel beinhalten kann;
R36 OH, O-(C₁-C₆)-Alkyl, O-(C₀-C₈)-Alkylen-aryl, CON(R51)(R52), N(R53)(R54), 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, J, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, O-(C₀-C₈)-Alkylen-aryl, (C₀-C₈)-Alkylen-aryl, N(R55)(R56), CO(C₁-C₆)-Alkyl, COO(R57) und S(O)ᵤ (R58) enthalten kann;
u 0, 1, 2;
R51, 52 unabhängig voneinander H, (C₁-C₈)-Alkyl, (C₂-C₆)-Alkenyl, (C₀-C₈)-Alkylen-aryl;
R53, R54 unabhängig voneinander H, (C₁-C₆)-Alkyl;
R55, R56 unabhängig voneinander H, (C₁-C₈)-Alkyl;
R57 H, (C₁-C₈)-Alkyl, (C₂-C₆)-Alkenyl, (C₀-C₈)-Alkylen-aryl;
R58 (C₁-C₆)-Alkyl, 5-10 gliedriges aromatisches Ringsystem, das 0-2 weitere Heteroatome aus der Gruppe Stickstoff, Sauerstoff und Schwefel enthalten und mit F, Cl, Br, CF₃, NO₂, CN, (C₁-C₈)-Alkyl, O-(C₁-C₈)-Alkyl substituiert sein kann;
sowie deren physiologisch verträgliche Salze.

2. Verbindungen der Formel I nach Anspruch 1,
worin bedeuten
R1 (C₁-C₈)-Alkyl, (C₁-C₄)-Alkoxy-(C₁-C₄)-alkyl, ein 3 bis 10-gliedriger mono-, bi- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, (C₀-C₂)-Alkylen-aryl, Oxo, CO(R3), CON(R4)(R5), Hydroxy, N(R7)CO(C₁-C₆)-Alkyl, N(R8)(R9) oder S0₂CH₃;
R3, R4, R5, R7, R8, R9 unabhängig voneinander H, (C₁-C₈)-Alkyl;
K eine Bindung, OCH₂, CH₂O, (C(R10)(R11))_{z}, C≡C;
z 1, 2, 3, 4;
R10, R11 unabhängig voneinander H, (C₁-C₈)-Alkyl;
E 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, N(R12)(R13), SO₂-CH₃, N(R16)CO(R17), N(R18)SO₂(R19), CO(R20) tragen und mono- oder bicyclisch sein kann;
R12, R13, R16, R18 unabhängig voneinander H, (C₁-C₈)-Alkyl;
R17, R19, R20 unabhängig voneinander H, (C₁-C₈)-Alkyl, Aryl;
X eine Bindung, -CH₂-CH₂-;
D, G entweder D ist N und G ist CH oder D ist CH und G ist N oder D und G sind gleichzeitig CH;
R2 OH, O-(C₁-C₆)-Alkyl, O-(C₁-C₄)-Alkoxy-(C₁-C₄)-alkyl, (C₁-C₆)-Alkyl;
n 0, 1, 2;
Q N(R24)(R25), einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit N(R24)(R25), F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃;
R26, R27, R28, R29, R30 unabhängig voneinander H, (C₁-C₆)-Alkyl;
A -(C(R31)(R32))ₘ-, in der 0-2 Glieder ersetzt sein können durch ein Element aus der Gruppe O, N(R33), CO;
m 0, 1, 2, 3, 4;
R31, R32, R33 unabhängig voneinander H, (C₁-C₆)-Alkyl, Aryl;
R24, R25 unabhängig voneinander H, (C₁-C₈)-Alkyl, -(CH₂)ₒ-R36, CO(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50);
o 0, 1, 2, 3, 4;
R36 OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆)-Alkyl, O-(C₀-C₂)-Alkylen-aryl, (C₀-C₂)-Alkylen-aryl und N(R55)(R56) enthalten kann;
R44, R45, R46, R48, R49, R50 unabhängig voneinander H, (C₁-C₆)-Alkyl ;
R55, R56 unabhängig voneinander H, (C₁-C₈)-Alkyl ;
sowie deren physiologisch verträgliche Salze.

3. Verbindungen der Formel I gemäß Anspruch 1 oder 2,
worin bedeuten:
R1 (C₁-C₈)-Alkyl, (C₁-C₄)-Alkoxy-(C₁-C₄)-alkyl, ein 3 bis 10-gliedriger mono-oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, Oxo, CO(R3), CON(R4)(R5), N(R7)CO(C₁-C₆)-Alkyl, oder SO₂CH₃;
R3, R4, R5, R7, R8, R9 unabhängig voneinander H, (C₁-C₈)-Alkyl;
K eine Bindung, OCH₂, CH₂O, (C(R10)(R11))_{z}, C≡C;
z 1, 2;
R10, R11 unabhängig voneinander H, (C₁-C₈)-Alkyl;
E 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R12)(R13), SO₂-CH₃, CO(R20) tragen kann;
R12, R13 unabhängig voneinander H,. (C₁-C₈)-Alkyl;
R20 unabhängig voneinander H, (C₁-C₈)-Alkyl;
X eine Bindung;
D, G gleichzeitig CH;
R2 OH, O-(C₁-C₆)-Alkyl, O-(C₁-C₄)-Alkoxy-(C₁-C₄)-alkyl, (C₁-C₆)-Alkyl;
n 0 oder 1;
Q N(R24)(R25), einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit N(R24)(R25), F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃;
R26, R27, R28, R29, R30 (C₁-C₆)-Alkyl;
A eine Gruppe der Formel -(C(R31)(R32))ₘ-, in der 1 Glied ersetzt sein kann durch ein Element aus der Gruppe O, N(R33);
m 0, 1, 3 oder 4;
R31, R32, R33 H;
R24, R25 unabhängig voneinander H, (C₁-C₈)-Alkyl, -(CH₂)ₒ-R36, CO(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50);
o 0, 1, 2, 3, 4;
R36 OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆)-Alkyl, O-(C₀-C₂)-Alkylen-aryl, (C₀-C₂)-Alkylen-aryl und N(R55)(R56) enthalten kann;
R44, R45, R46, R48, R49, R50 unabhängig voneinander H, (C₁-C₆)-Alkyl;
R55, R56 unabhängig voneinander H, (C₁-C₈)-Alkyl;
sowie deren physiologisch verträgliche Salze.

4. Verbindungen der Formel I nach Anspruch 1
Worin die Reste R1 und R2, der Index n und die Gruppen K, E, X, D=G, Q und A die folgenden Bedeutungen aufweisen
R1 H, (C₁-C₈)-Alkyl, (C₁-C₄)-Alkoxy-(C₁-C₄)-alkyl, ein 3 bis 8-gliedriger monocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, (C₁-C₆)-Alkyl, O-(C₁-C₈)-Alkyl, (C₁-C₄)-Alkoxy-(C₁-C₄)-alkyl ;
K O, eine Bindung, C≡C, CO, OCH₂, OCH₂CH₂ ;
E 5-6 gliedrige monocyclische bivalente carbo- oder heterocyclische Ringstruktur mit 0-2 Heteroatomen aus der Gruppe N, O und S, die optional Substituenten aus der Gruppe H, F, Cl, Br, Oxo, O-(C₁-C₆)-Alkyl, O-(C₁-C₄)-Alkoxy-(C₁-C₄)-alkyl, (C₁-C₆)-Alkyl tragen kann;
X eine Bindung, CH₂CH₂, CH₂ CH₂ CH₂, OCH₂CH₂;
D=G CH=CH, CH=N, N=CH;
R2 OH, O-(C₁-C₆)-Alkyl, (C₁-C₆)-Alkyl;
n 0, 1, 2;
Q eine Gruppe der Formel N(R24)(R25), ein stickstoffhaltiger 4 bis 8-gliedriger Ring der außer dem Stickstoffatom noch weitere 0-2 Heteroatome aus der Gruppe Sauerstoff, Stickstoff und Schwefel enthalten kann und wobei das Ringsystem zusätzlich substituiert sein kann mit N(R24)(R25), F, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), N(R30)CO(C₁-C₆)-Alkyl oder SO₂CH₃;
R24, R25 unabhängig voneinander H, (C₁-C₈)-Alkyl, (C₁-C₄)-Alkoxy-(C₁-C₄)-alkyl, CO(C₁-C₈)-Alkyl, oder R24 und R25 bilden zusammen mit dem Stickstoffatom, an das sie gebunden sind, einen 4 bis 8-gliedrigen monocyclischen Ring, welcher ausser dem Stickstoffatom 0 bis 1 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl, N(R48)CO(C₁-C₆)-Alkyl oder N(R49)(R50);
R48,R49,R50 unabhängig voneinander H, (C₁-C₆)-Alkyl;
R49 und R50 optional mit dem Stickstoffatom, an das sie gebunden sind, einen 5-6 gliedrigen Ring, welcher ausser dem Stickstoffatom noch 0-1 weitere Heteroatome aus der Gruppe N-(C1-C6)-Alkyl, Sauerstoff und Schwefel beinhalten kann;
R26, R30 unabhängig voneinander H, (C₁-C₆)-Alkyl;
A eine Gruppe der Formel -(C(R31)(R32))ₘ-, in der 1 Glied ersetzt sein kann durch ein Element aus der Gruppe O, N(R33);
m 0, 1, 3;
R31, R32, R33 unabhängig voneinander H, (C₁-C₆)-Alkyl, Aryl.

5. Verbindungen der Formel I, gemäß einem der Ansprüche 1 bis 4, **dadurch gekennzeichnet, dass**
D und G jeweils CH bedeuten.

6. Verbindungen der Formel I, gemäß einem der Ansprüche 1 bis 3 oder 5,
**dadurch gekennzeichnet, dass**
A eine Gruppe der Formel -(C(R31)(R32))ₘ-, in der 0-2 Glieder ersetzt sein können durch ein Element aus der Gruppe O, N(R33), CO bedeutet;
wobei
m 1, 2, 3, 4 ist;
und
R31, R32, R33 unabhängig voneinander H, (C₁-C₆)-Alkyl, Aryl sind;
und
Q N(R24)(R25) bedeutet;
wobei
R24, R25 unabhängig voneinander H, (C₁-C₈)-Alkyl, -(CH₂)ₒ-R36, CO-(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50) bedeuten;
o 0, 1, 2, 3, 4 ist;
R36 OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆)-Alkyl, O-(C₀-C₂)-Alkylen-aryl, (C₀-C₂)-Alkylen-aryl und N(R55)(R56) enthalten kann, bedeutet;
R44, R45, R46, R48, R49, R50 unabhängig voneinander H, (C₁-C₆)-Alkyl sind;
und
R55, R56 unabhängig voneinander H, (C₁-C₈)-Alkyl sind.

7. Verbindungen der Formel I nach einem der Ansprüche 1 bis 3 oder 5,
**dadurch gekennzeichnet, dass**
A eine Gruppe der Formel -(C(R31)(R32))ₘ-, in der 0-2 Glieder ersetzt sein können durch ein Element aus der Gruppe O, N(R33), CO; bedeutet;
wobei
m 0, 1, 2, 3, 4 ist;
und
R31, R32, R33 unabhängig voneinander H, (C₁-C₆)-Alkyl, Aryl; sind;
und
Q einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃; bedeutet;
wobei
R26, R27, R28, R29, R30 unabhängig voneinander H, (C₁-C₆)-Alkyl bedeuten.

8. Verbindungen nach einem der Ansprüche 1 bis 3 oder 5, **dadurch gekennzeichnet, dass**
A eine Gruppe der Formel -(C(R31)(R32))ₘ-, in der 0-2 Glieder ersetzt sein können durch ein Element aus der Gruppe O, N(R33), CO;
wobei
m 0, 1, 2, 3, 4 ist;
und
R31, R32, R33 unabhängig voneinander H, (C₁-C₆)-Alkyl, Aryl sind;
und
Q einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert ist mit N(R24)(R25) und zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃, bedeutet;
wobei
R26, R27, R28, R29, R30 unabhängig voneinander H, (C₁-C₆)-Alkyl bedeuten;
und
R24, R25 unabhängig voneinander H, (C₁-C₈)-Alkyl, -(CH₂)ₒ-R36, CO-(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), CON(R45)(R46), Hydroxy, N(R48)CO(C₁-C₆)-Alkyl oder N(R49)(R50) bedeuten;
o 0, 1, 2, 3, 4 ist;
R36 OH, 5-10 gliedriger mono- oder bicyclischer Ring, der ein oder mehrere Heteroatome aus der Gruppe N, O und S enthalten kann und der 5-10 gliedrige Ring weitere Substituenten wie F, Cl, Br, OH, CF₃, Oxo, O-(C₁-C₆)-Alkyl, (C₁-C₆)-Alkyl, O-(C₀-C₂)-Alkylen-aryl, (C₀-C₂)-Alkylen-aryl und N(R55)(R56) enthalten kann, bedeutet;
R44, R45, R46, R48, R49, R50 unabhängig voneinander H, (C₁-C₆)-Alkyl sind;
und
R55, R56 unabhängig voneinander H, (C₁-C₈)-Alkyl sind.

9. Verbindungen der Formel I gemäß einem der Ansprüche 1 bis 3, worin bedeuten:
R1 (C₁-C₈)-Alkyl, (C₁-C₄)-Alkoxy-(C₁-C₄)-alkyl, ein 3 bis 10-gliedriger mono-oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, Oxo, CO(R3), CON(R4)(R5), N(R7)CO(C₁-C₆)-Alkyl, oder SO₂CH₃ ;
R3, R4, R5, R7, R8, R9 unabhängig voneinander H, (C₁-C₈)-Alkyl ;
K eine Bindung, OCH₂, CH₂O, (C(R10)(R11))_{z}, C≡C ;
z 1, 2 ;
R10, R11 unabhängig voneinander H, (C₁-C₈)-Alkyl ;
E 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R12)(R13), SO₂-CH₃, CO(R20) tragen kann;
R12, R13 unabhängig voneinander H, (C₁-C₈)-Alkyl;
R20 unabhängig voneinander H, (C₁-C₈)-Alkyl;
X eine Bindung;
D, G gleichzeitig CH;
R2 O-(C₁-C₆)-Alkyl, (C₁-C₆)-Alkyl;
n 0 oder 1;
Q N(R24)(R25), einen 3 bis 8-gliedrigen monocyclischen Ring welcher 0 bis 3 Heteroatome beinhalten kann, ausgewählt aus der Gruppe Sauerstoff, Stickstoff und Schwefel, wobei das Ringsystem zusätzlich substituiert sein kann mit N(R24)(R25), F, Cl, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), N(R30)CO(C₁-C₆)-Alkyl oder SO₂CH₃;
R26, R30 (C₁-C₆)-Alkyl;
A eine Gruppe der Formel -(C(R31)(R32))ₘ-, in der 1 Glied ersetzt sein kann durch ein Element aus der Gruppe O, N(R33);
m 0, 1, 3 oder 4;
R31, R32, R33 H;
R24, R25 unabhängig voneinander H, (C₁-C₈)-Alkyl, -(CH₂)ₒ-R36, CO(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, (C₁-C₆)-Alkyl, O-(C₁-C₈)-Alkyl, Oxo, CO(R44), N(R48)CO(C₁-C₆)-Alkyl oder N(R49)(R50);
o 0, 1, 2, 3, 4;
R36 5-8 gliedriger monocyclischer Ring, der 0-2 Heteroatome aus der Gruppe N, O und S enthalten kann und der 5-8 gliedrige Ring weitere Substituenten wie F, Oxo, O-(C₁-C₆)-Alkyl, (C₁-C₆)-Alkyl, (C₀-C₂)-Alkylen-aryl und N(R55)(R56) enthalten kann;
R44,R48,R49,R50 unabhängig voneinander H, (C₁-C₆)-Alkyl;
R55, R56 unabhängig voneinander H, (C₁-C₈)-Alkyl;
sowie deren physiologisch verträgliche Salze.

10. Verbindungen der Formel I gemäß einem der Ansprüche 1 bis 3, 5 oder 6, **dadurch gekennzeichnet, dass**
A eine Gruppe der Formel -(C(R31)(R32))ₘ-, in der 1 Glied ersetzt sein können durch ein Element aus der Gruppe O, N(R33) bedeutet;
wobei
m 1, 3 oder 4 ist;
und
R31, R32, R33 H sind;
und Q N(R24)(R25) bedeutet;
wobei
R24, R25 unabhängig voneinander H, (C₁-C₈)-Alkyl, -(CH₂)ₒ-R36, CO(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, (C₁-C₆)-Alkyl, O-(C₁-C₈)-Alkyl, Oxo, CO(R44), N(R48)CO(C₁-C₆)-Alkyl oder N(R49)(R50);
o 0, 1, 2, 3, 4 ist;
R36 OH, 5-8 gliedriger monocyclischer Ring, der 0-2 Heteroatome aus der Gruppe N, O und S enthalten kann und der 5-8 gliedrige Ring weitere Substituenten wie F, Oxo, O-(C₁-C₆)-Alkyl, (C₁-C₆)-Alkyl, (C₀-C₂)-Alkylen-aryl und N(R55)(R56) enthalten kann, bedeutet;
R44, R48, R49, R50 unabhängig voneinander H, (C₁-C₆)-Alkyl sind;
R55, R56 unabhängig voneinander H, (C₁-C₈)-Alkyl sind.

11. Verbindungen der Formel I nach einem der Ansprüche 1 bis 3, 5 oder 7, worin
A eine Gruppe der Formel -(C(R31)(R32))ₘ-, in der 1 Glied ersetzt sein können durch ein Element aus der Gruppe O, N(R33) bedeutet;
wobei
m 0 oder 1 ist;
und
R31, R32, R33 H sind;
und
Q einen 5 bis 7-gliedrigen monocyclischen Ring welcher 1 oder 2 Stickstoffatome enthält, wobei das Ringsystem zusätzlich substituiert sein kann mit (C₁-C₆)-Alkyl, (C₁-C₄)-Alkoxy-(C₁-C₄)-alkyl, (C₀-C₈)-Alkylen-aryl; bedeutet.

12. Verbindungen der Formel I nach einem der Ansprüche 1 bis 3, 5 oder 8, **dadurch gekennzeichnet, dass**
A eine Gruppe der Formel -(C(R31)(R32))ₘ-, in der 1 Glied ersetzt sein kann durch ein Element aus der Gruppe O, N(R33) bedeutet;
wobei
m 0 oder 1 ist;
und
R31, R32, R33 H sind;
Q einen 3 bis 8-gliedrigen monocyclischen Ring welcher 0 bis 1 Heteroatom beinhalten kann, ausgewählt aus der Gruppe Sauerstoff, Stickstoff und Schwefel, wobei das Ringsystem zusätzlich substituiert ist mit N(R24)(R25) und zusätzlich substituiert sein kann mit mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R26), CON(R27)(R28), Hydroxy, Hydroxy-(C₁-C₄)-alkyl, COO(R29), N(R30)CO(C₁-C₆)-Alkyl oder SO₂CH₃, bedeutet;
wobei
R26, R27, R28, R29, R30 (C₁-C₆)-Alkyl bedeuten;
und
R24, R25 unabhängig voneinander H, (C₁-C₈)-Alkyl, -(CH₂)ₒ-R36, CO-(C₁-C₈)-Alkyl, oder R24 und R25 bilden zusammen mit dem Stickstoffatom, an das sie gebunden sind, einen 4 bis 10-gliedrigen mono-, bi- oder spirocyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, (C₁-C₆)-Alkyl, O-(C₁-C₈)-Alkyl, Oxo, CO(R44), N(R48)CO(C₁-C₆)-Alkyl oder N(R49)(R50) bedeuten;
o 0, 1, 2, 3, 4 ist;
R36 5-8 gliedriger monocyclischer Ring, der 0-2 Heteroatome aus der Gruppe N, O und S enthalten kann und der 5-8 gliedrige Ring weitere Substituenten wie F, Oxo, O-(C₁-C₆)-Alkyl, (C₁-C₆)-Alkyl, (C₀-C₂)-Alkylen-aryl und N(R55)(R56) enthalten kann, bedeutet.

13. Verfahren zur Herstellung von Verbindungen nach einem der Ansprüche 1 bis 12, deren Gruppierung A-Q über ein Stickstoffatom mit dem Cyclohexylen-Ring verknüpft ist, umfassend die Schritte
a) Herstellung einer Verbindung der Formel II durch Umsetzung von Cyclohexadion mit 1,2-Ethylenglycol 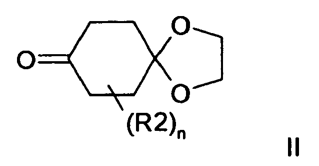
b) Umsetzung der Verbindung der Formel II mit 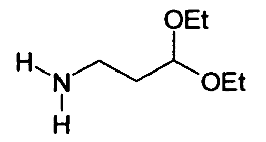 zu einer Verbindung der Formel III 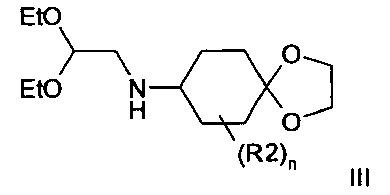
c) Kupplung der Verbindung der Formel III mit einem primären Amin der Formel 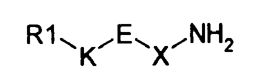 und anschließendes Entschützen des Acetals, wobei unter gleichzeitiger Cyclisierung eine Verbindung der Formel IV erhalten wird 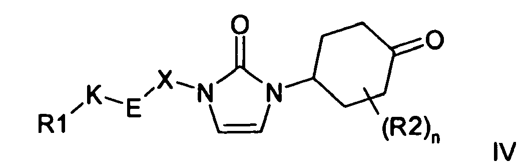
d) Umsetzung mit einem sekundären Amin, wobei Verbindungen der Formel I erhalten werden, deren Gruppierung A-Q über ein Stickstoffatom mit dem Cyclohexylen-Ring verknüpft ist

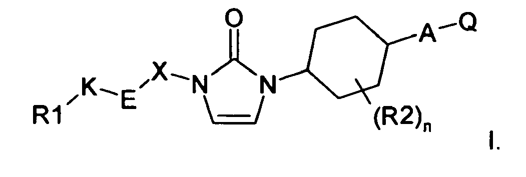

14. Arzneimittel enthaltend eine oder mehrere der Verbindungen gemäß einem der Ansprüche 1 bis 12.

15. Arzneimittel enthaltend eine oder mehrere der Verbindungen gemäß einem der Ansprüche 1 bis 12 und einen oder mehrere anorektische Wirkstoffe.

16. Verbindungen der Formel I gemäß einem der Ansprüche 1 bis 12 zur Anwendung als Medikament zur Prophylaxe oder Behandlung der Obesitas.

17. Verbindungen der Formel I gemäß einem der Ansprüche 1 bis 12 zur Anwendung als Medikament zur Prophylaxe oder Behandlung des Typ II Diabetes.

18. Verbindungen der Formel I gemäß einem der Ansprüche 1 bis 12 in Kombination mit mindestens einem weiteren anorektischen Wirkstoff zur Anwendung als Medikament zur Prophylaxe oder Behandlung der Obesitas.

19. Verbindungen der Formel I gemäß einem der Ansprüche 1 bis 12 in Kombination mit mindestens einem weiteren anorektischen Wirkstoff zur Anwendung als Medikament zur Prophylaxe oder Behandlung der des Typ II Diabetes.

20. Verfahren zur Herstellung eines Arzneimittels enthaltend eine oder mehrere der Verbindungen der Formel I gemäß einem der Ansprüche 1 bis 12, **dadurch gekennzeichnet, dass** der Wirkstoff mit einem pharmazeutisch geeigneten Träger vermischt wird und diese Mischung in eine für die Verabreichung geeignete Form gebracht wird.

21. Verwendung der Verbindungen der Formel I gemäß einem der Ansprüche 1 bis 12 zur Herstellung eines Medikaments zur Gewichtsreduktion bei Säugetieren.

22. Verwendung der Verbindungen der Formel I gemäß einem der Ansprüche 1 bis 12 zur Herstellung eines Medikaments zur Prophylaxe oder Behandlung der Obesitas.

23. Verwendung der Verbindungen der Formel I gemäß einem der Ansprüche 1 bis 12 zur Herstellung eines Medikaments zur Prophylaxe oder Behandlung des Typ II Diabetes.

## Claims

1. A compound of the formula I, 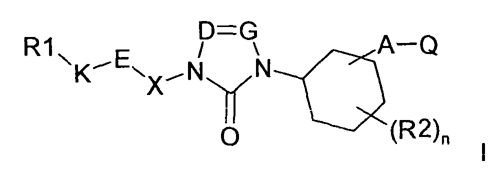 in which the meanings are
R1 H, (C₁-C₈)-alkyl, (C₁-C₄)-alkoxy-(C₁-C₄)-alkyl, (C₃-C₈)-alkenyl, (C₃-C₈)-alkynyl, a 3 to 10-membered mono-, bi- or spirocyclic ring which may include 0 to 4 heteroatoms selected from the group of oxygen, nitrogen and sulfur, where the ring system may additionally be substituted by F, Cl, Br, CF₃, NO₂, CN, (C₁₋C₆)-alkyl, O-(C₁-C₈)-alkyl, S-(C₁-C₈)-alkyl, (C₁-C₄)-alkoxy-(C₁-C₄)-alkyl, (C₀-C₈)-alkylene-aryl, oxo, CO(R3), CON(R4)(R5), hydroxy, hydroxy-(C₁-C₄)-alkyl, COO(R6), N(R7)CO(C₁-C₆)-alkyl, N(R8)(R9) or SO₂CH₃;
R3, R4, R5, R6, R7, R8, R9, independently of one another H, (C₁-C₆)-alkyl;
K a group of the formula -(CR10R11)_{z}-, in which one or more -(CR10R11)- groups may be replaced by Z, a bond, C≡C, C=C;
Z O, CO, N(R59), S, SO, SO₂;
R10, R11 independently of one another H, (C₁-C₈)-alkyl, hydroxy-(C₁-C₄)-alkyl, hydroxy, (C₁-C₄)-alkoxy-(C₁-C₄)-alkyl, where R10 and R11 in the z groups may in each case have the same or different meanings;
z 1, 2, 3, 4, 5, 6;
R59 H, (C₁-C₈)-alkyl;
E 3-14 membered bivalent carbo- or heterocyclic ring structure having 0-4 heteroatoms from the group of N, O and S, which may optionally have substituents from the group of H, F, Cl, Br, I, OH, CF₃, NO₂, CN, OCF₃, oxo, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O-(C₃-C₈)-cycloalkenyl, (C₂-C₆)-alkynyl, (C₀-C₈)-alkylene-aryl, O-(C₀-C₈)-alkylene-aryl, S-aryl, N(R12)(R13), SO₂-CH₃, COOH, COO-(C₁-C₆)-alkyl, CON(R14)(R15), N(R16)CO(R17), N(R18)SO₂(R19), CO(R20) and be mono- or bicyclic;
R12, R13, R14, R15, R16, R18 independently of one another H, (C₁-C₈)-alkyl;
or
R12 and R13, R14 and R15 independently of one another, optionally together with the nitrogen atom to which they are bonded, a 5-6 membered ring which, apart from the nitrogen atom, may also include 0-1 further heteroatoms from the group of N-(C₁-C₆)-alkyl, oxygen and sulfur;
R17, R19, R20 independently of one another H, (C₁-C₈)-alkyl, aryl;
X a bond, a group of the formula -(CR21 R22)_{Y}-, in which one or more -(CR21 R22)- groups may be replaced by Y;
Y O, CO, N(R23), S, SO, SO₂;
R21, R22 independently of one another H, (C₁-C₄)-alkyl, where R21 and R22 in the y groups may in each case have the same or different meanings;
y 1, 2, 3, 4, 5, 6;
R23 H, (C₁-C₈)-alkyl;
D, G CH or N;
R2 OH, O-(C₁-C₆)-alkyl, O-(C₁-C₄)-alkoxy-(C₁-C₄)-alkyl, (C₁-C₆)-alkyl;
n 0, 1, 2, 3, 4;
Q N(R24)(R25), a 3 to 8-membered ring which may include 0 to 3 heteroatoms selected from the group of oxygen, nitrogen and sulfur, where the ring system may additionally be substituted by (C₀-C₄)-alkylene-N(R24)(R25), F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), CON(R27)(R28), hydroxy, hydroxy-(C₁-C₄)-alkyl, COO(R29), N(R30)CO(C₁-C₆)-alkyl or SO₂CH₃;
R26, R27, R28, R29, R30 independently of one another H, (C₁-C₆)-alkyl ;
A a group of the formula -(C(R31)(R32))ₘ-, in which 0-2 members may be replaced by an element from the group of O, S, N(R33), CO, SO₂ ;
m 0, 1, 2, 3, 4, 5 ;
R31, R32, R33 independently of one another H, (C₁-C₆)-alkyl, aryl;
R24, R25 independently of one another H, (C₁-C₈)-alkyl, -(CR34R35)ₒ-R36, (C₁-C₄)-alkoxy-(C₁-C₄)-alkyl, (C₃-C₈)-alkenyl, (C₃-C₈)-alkynyl, CO-(C₁-C₈)-alkyl, -CO-(CH₂)ₒ -R36, CO(C(R37)(R38))qN(R39)(R40), CO(C(R41)(R42))ₛO(R43); or R24 and R25 form together with the nitrogen atom to which they are bonded a 4 to 10-membered mono-, bi- or spirocyclic ring which, apart from the nitrogen atom, may include 0 to 4 additional heteroatoms selected from the group of oxygen, nitrogen and sulfur, where the heterocyclic ring system may additionally be substituted by F, Cl, Br, CF₃, NO₂, CN, (C₁-C₆)-alkyl, O-(C₁-C₈)-alkyl, (C₁-C₄)-alkoxy-(C₁-C₄)-alkyl, hydroxy-(C₁-C₄)-alkyl, (C₀-C₈)-alkylene-aryl, oxo, CO(R44), CON(R45)(R46), hydroxy, COO(R47), N(R48)CO(C₁-C₆)-alkyl, N(R49)(R50) or SO₂CH₃;
o 0, 1, 2, 3, 4, 5, 6;
q, s independently of one another 0, 1, 2, 3, 4;
R34, R35 independently of one another H, (C₁-C₈)-alkyl, hydroxy-(C₁-C₄)-alkyl, OH, (C₁-C₄)-alkoxy-(C₁-C₄)-alkyl;
R37, R38, R39, R40, R41, R42, R43, R44, R45, R46, R47, R48, R49, R50 independently of one another H, (C₁-C₆)-alkyl;
R39 and R40, R45 and R46, R49 and R50 independently of one another, optionally together with the nitrogen atom to which they are bonded a 5-6 membered ring which, apart from the nitrogen atom, may also include 0-1 further heteroatoms from the group of N-(C₁-C₆)-alkyl, oxygen and sulfur;
R36 OH, O-(C₁-C₆)-alkyl, O-(C₀-C₈)-alkylene-aryl, CON(R51)(R52), N(R53)(R54), 3-12 membered mono-, bi- or spirocyclic ring which may comprise one or more heteroatoms from the group of N, O and S, and the 3-12 membered ring may comprise further substituents such as F, Cl, Br, I, OH, CF₃, NO₂, CN, OCF₃, oxo, O-(C₁-C₆)-alkyl, (C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O-(C₃-C₈)-cycloalkenyl, (C₂-C₆)-alkynyl, O-(C₀-C₈)-alkylene-aryl, (C₀-C₈)-alkylene-aryl, N(R55)(R56), CO(C₁-C₆)-alkyl, COO(R57) and S(O)ᵤ (R58);
u 0, 1, 2;
R51, 52 independently of one another H, (C₁-C₈)-alkyl, (C₂-C₆)-alkenyl, (C₀-C₈)-alkylene-aryl;
R53, R54 independently of one another H, (C₁-C₆)-alkyl;
R55, R56 independently of one another H, (C₁-C₈)-alkyl;
R57 H, (C₁-C₈)-alkyl, (C₂-C₆)-alkenyl, (C₀-C₈)-alkylene-aryl ;
R58 (C₁-C₆)-alkyl, 5-10 membered aromatic ring system which may comprise 0-2 further heteroatoms from the group of nitrogen, oxygen and sulfur and be substituted by F, Cl, Br, CF₃, NO₂, CN, (C₁-C₈)-alkyl, O-(C₁-C₈)-alkyl ;
and the physiologically tolerated salts thereof.

2. A compound of the formula I as claimed in claim 1,
in which the meanings are
R1 (C₁-C₈)-alkyl, (C₁-C₄)-alkoxy-(C₁-C₄)-alkyl, a 3 to 10-membered mono-, bi- or spirocyclic ring which may include 0 to 3 heteroatoms selected from the group of oxygen, nitrogen and sulfur, where the ring system may additionally be substituted by F, Cl, Br, CF₃, CN, (C₁-C₆)-alkyl, O-(C₁-C₈)-alkyl, (C₀-C₂)-alkylene-aryl, oxo, CO(R3), CON(R4)(R5), hydroxy, N(R7)CO(C₁-C₆)-alkyl, N(R8)(R9) or SO₂CH₃;
R3, R4, R5, R7, R8, R9 independently of one another H, (C₁-C₈)-alkyl;
K a bond, OCH₂, CH₂O, (C(R10)(R11))_{z}, C≡C;
z 1, 2, 3, 4;
R10, R11 independently of one another H, (C₁-C₈)-alkyl;
E 3-8 membered bivalent carbo- or heterocyclic ring structure having 0-4 heteroatoms from the group of N, O and S, which may optionally have substituents from the group of H, F, Cl, Br, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, N(R12)(R13), SO₂-CH₃, N(R16)CO(R17), N(R18)SO₂(R19), CO(R20) and be mono- or bicyclic;
R12, R13, R16, R18 independently of one another H, (C₁-C₈)-alkyl;
R17, R19, R20 independently of one another H, (C₁-C₈)-alkyl, aryl;
X a bond, -CH₂-CH₂- ;
D, G either D is N and G is CH or D is CH and G is N or D and G are both CH;
R2 OH, O-(C₁-C₆)-alkyl, O-(C₁-C₄)-alkoxy-(C₁-C₄)-alkyl, (C₁-C₆)-alkyl;
n 0, 1, 2;
Q N(R24)(R25), a 3 to 8-membered monocyclic ring which may include 0 to 3 heteroatoms selected from the group of oxygen, nitrogen and sulfur, where the ring system may be additionally substituted by N(R24)(R25), F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), CON(R27)(R28), hydroxy, hydroxy-(C₁-C₄)-alkyl, COO(R29), N(R30)CO(C₁-C₆)-alkyl or SO₂CH₃;
R26, R27, R28, R29, R30 independently of one another H, (C₁-C₆)-alkyl;
A -(C(R31)(R32))ₘ- in which 0-2 members may be replaced by an element from the group of O, N(R33), CO;
m 0, 1, 2, 3, 4;
R31, R32, R33 independently of one another H, (C₁-C₆)-alkyl, aryl;
R24, R25 independently of one another H, (C₁-C₈)-alkyl, -(CH₂)ₒ-R36, CO-(C₁-C₈)-alkyl, or R24 and R25 form together with the nitrogen atom to which they are bonded a 4 to 10-membered, mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, Cl, (C₁-C₆)-alkyl, O-(C₁-C₈)-alkyl, oxo, CO(R44), CON(R45)(R46), hydroxy, N(R48)CO(C₁-C₆)-alkyl or N(R49)(R50);
o 0, 1, 2, 3, 4;
R36 OH, 5-10 membered mono- or bicyclic ring which may comprise one or more heteroatoms from the group of N, O and S, and the 5-10 membered ring may comprise further substituents such as F, Cl, Br, OH, CF₃, oxo, O-(C₁-C₆)-alkyl, (C₁-C₆)-alkyl, O-(C₀-C₂)-alkylene-aryl, (C₀-C₂)-alkylene-aryl and N(R55)(R56);
R44, R45, R46, R48, R49, R50 independently of one another H, (C₁-C₆)-alkyl;
R55, R56 independently of one another H, (C₁-C₈)-alkyl;
and the physiologically tolerated salts thereof.

3. A compound of the formula I as claimed in claim 1 or 2,
in which the meanings are:
R1 (C₁-C₈)-alkyl, (C₁-C₄)-alkoxy-(C₁-C₄)-alkyl, a 3 to 10-membered mono- or bicyclic ring which may include 0 to 2 heteroatoms selected from the group of oxygen, nitrogen and sulfur, where the ring system may additionally be substituted by F, Cl, Br, CF₃, CN, (C₁-C₆)-alkyl, O-(C₁-C₈)-alkyl, oxo, CO(R3), CON(R4)(R5), N(R7)CO(C₁-C₆)-alkyl, or SO₂CH₃;
R3, R4, R5, R7, R8, R9 independently of one another H, (C₁-C₈)-alkyl;
K a bond, OCH₂, CH₂O, (C(R10)(R11))_{z}, C≡C;
z 1,2;
R10, R11 independently of one another H, (C₁-C₈)-alkyl;
E 5-7 membered bivalent carbo- or heterocyclic ring structure having 0-2 heteroatoms from the group of N, O and S, which may optionally have substituents from the group of H, F, Cl, Br, OH, CF₃, NO₂, OCF₃, O-(C₁-C₆)-alkyl, (C₁-C₆)-alkyl, (C₂-C₆)-alkenyl, N(R12)(R13), SO₂-CH₃, CO(R20);
R12, R13 independently of one another H, (C₁-C₈)-alkyl;
R20 independently of one another H, (C₁-C₈)-alkyl;
X a bond;
D, G both CH;
R2 OH, O-(C₁-C₆)-alkyl, O-(C₁-C₄)-alkoxy-(C₁-C₄)-alkyl, (C₁-C₆)-alkyl;
n 0 or 1;
Q N(R24)(R25), a 3 to 8-membered monocyclic ring which may include 0 to 3 heteroatoms selected from the group of oxygen, nitrogen and sulfur, where the ring system may additionally be substituted by N(R24)(R25), F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), CON(R27)(R28), hydroxy, hydroxy-(C₁-C₄)-alkyl, COO(R29), N(R30)CO(C₁-C₆)-alkyl or SO₂CH₃ ;
R26, R27, R28, R29, R30 (C₁-C₆)-alkyl;
A a group of the formula -(C(R31)(R32))ₘ- in which 1 member may be replaced by an element from the group of O, N(R33);
m 0, 1, 3 or 4;
R31, R32, R33 H;
R24, R25 independently of one another H, (C₁-C₈)-alkyl, -(CH₂)₀-R36, CO(C₁-C₈)-alkyl, or R24 and R25 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, Cl, (C₁-C₆)-alkyl, O-(C₁-C₈)-alkyl, oxo, CO(R44), CON(R45)(R46), hydroxy, N(R48)CO(C₁-C₆)-alkyl or N(R49)(R50);
o 0, 1, 2, 3, 4 ;
R36 OH, 5-10 membered mono- or bicyclic ring which may comprise one or more heteroatoms from the group of N, O and S, and the 5-10 membered ring may comprise further substituents such as F, Cl, Br, OH, CF₃, oxo, O-(C₁-C₆)-alkyl, (C₁-C₆)-alkyl, O-(C₀-C₂)-alkylene-aryl, (C₀-C₂)-alkylene-aryl and N(R55)(R56);
R44, R45, R46, R48, R49, R50 independently of one another H, (C₁-C₆)-alkyl;
R55, R56 independently of one another H, (C₁-C₈)-alkyl;
and the physiologically tolerated salts thereof.

4. A compound of the formula I as claimed in claim 1,
in which the radicals R1 and R2, index n and the groups K, E, X, D=G, Q and A have the following meanings
R1 H, (C₁-C₈)-alkyl, (C₁-C₄)-alkoxy-(C₁-C₄)-alkyl, a 3 to 8-membered monocyclic ring which may include 0 to 3 heteroatoms selected from the group of oxygen, nitrogen and sulfur, where the ring system may additionally be substituted by F, Cl, Br, (C₁-C₆)-alkyl, O-(C₁-C₈)-alkyl, (C₁-C₄)-alkoxy-(C₁-C₄)-alkyl;
K O, a bond, C≡C, CO, OCH₂, OCH₂CH₂;
E 5-6 membered monocyclic bivalent carbo- or heterocyclic ring structure having 0-2 heteroatoms from the group of N, O and S, which may optionally have substituents from the group of H, F, Cl, Br, oxo, O-(C₁-C₆)-alkyl, O-(C₁-C₄)-alkoxy-(C₁-C₄)-alkyl, (C₁-C₆)-alkyl;
X a bond, CH₂CH₂, CH₂CH₂CH₂, OCH₂CH₂;
D=G CH=CH, CH=N, N=CH;
R2 OH, O-(C₁-C₆)-alkyl, (C₁-C₆)-alkyl;
n 0, 1, 2;
Q a group of the formula N(R24)(R25), a nitrogen atom containing 4 to 8-membered ring which, apart from the nitrogen atom, may also comprise a further 0-2 heteroatoms from the group of oxygen, nitrogen and sulfur, and where the ring system may additionally be substituted by N(R24)(R25), F, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), N(R30)CO(C₁-C₆)-alkyl or S0₂CH₃;
R24, R25 independently of one another H, (C₁-C₈)-alkyl, (C₁-C₄)-alkoxy-(C₁-C₄)-alkyl, CO(C₁-C₈)-alkyl, or R24 and R25 form together with the nitrogen atom to which they are bonded a 4 to 8-membered monocyclic ring which, apart from the nitrogen atom, may include 0 to 1 additional heteroatoms selected from the group of oxygen, nitrogen and sulfur, where the heterocyclic ring system may additionally be substituted by (C₁-C₆)-alkyl, N(R48)CO(C₁-C₆)-alkyl or N(R49)(R50);
R48, R49, R50 independently of one another H, (C₁-C₆)-alkyl;
R49 and R50 optionally with the nitrogen atom to which they are bonded a 5-6 membered ring which, apart from the nitrogen atom, may also include 0-1 further heteroatoms from the group of N-(C1-C6)-alkyl, oxygen and sulfur;
R26, R30 independently of one another H, (C₁-C₆)-alkyl;
A a group of the formula -(C(R31)(R32)ₘ-, in which 1 member may be replaced by an element from the group of O, N(R33);
m 0, 1, 3;
R31, R32, R33 independently of one another H, (C₁-C₆)-alkyl, aryl.

5. A compound of the formula I as claimed in any of claims 1 to 4, wherein
D and G are each CH.

6. A compound of the formula I as claimed in any of claims 1 to 3 or 5, wherein
A is a group of the formula -(C(R31)(R32))ₘ- in which 0-2 members may be replaced by an element from the group of O, N(R33), CO;
where
m is 1, 2, 3, 4;
and
R31, R32, R33 are independently of one another H, (C₁-C₆)-alkyl, aryl;
and
Q is N(R24)(R25);
where
R24, R25 are independently of one another H, (C₁-C₈)-alkyl, -(CH₂)ₒ-R36, CO-(C₁-C₈)-alkyl, or R24 and R25 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, Cl, (C₁-C₆)-alkyl, O-(C₁-C₈)-alkyl, oxo, CO(R44), CON(R45)(R46), hydroxy, N(R48)CO(C₁-C₆)-alkyl or N(R49)(R50);
o is 0, 1, 2, 3, 4;
R36 is OH, 5-10 membered mono- or bicyclic ring which may comprise one or more heteroatoms from the group of N, O and S, and the 5-10 membered ring may comprise further substituents such as F, Cl, Br, OH, CF₃, oxo, O-(C₁-C₆)-alkyl, (C₁-C₆)-alkyl, O-(C₀-C₂)-alkylene-aryl, (C₀-C₂)-alkylene-aryl and N(R55)(R56);
R44, R45, R46, R48, R49, R50 are independently of one another H, (C₁-C₆)-alkyl;
and
R55, R56 are independently of one another H, (C₁-C₈)-alkyl.

7. A compound of the formula I as claimed in any of claims 1 to 3 or 5, wherein
A is a group of the formula -(C(R31)(R32))ₘ- in which 0-2 members may be replaced by an element from the group of O, N(R33), CO;
where
m is 0, 1, 2, 3, 4;
and
R31, R32, R33 are independently of one another H, (C₁-C₆)-alkyl, aryl;
and
Q is a 3 to 8-membered monocyclic ring which may include 0 to 3 heteroatoms selected from the group of oxygen, nitrogen and sulfur, where the ring system may additionally be substituted by F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), CON(R27)(R28), hydroxy, hydroxy-(C₁-C₄)-alkyl, COO(R29), N(R30)CO(C₁-C₆)-alkyl or SO₂CH₃;
where
R26, R27, R28, R29, R30 are independently of one another H, (C₁-C₆)-alkyl.

8. A compound as claimed in any of claims 1 to 3 or 5, wherein
A is a group of the formula -(C(R31)(R32))ₘ- in which 0-2 members may be replaced by an element from the group of O, N(R33), CO;
where
m is 0, 1, 2, 3, 4;
and
R31, R32, R33 are independently of one another H, (C₁-C₆)-alkyl, aryl;
and
Q is a 3 to 8-membered monocyclic ring which may include 0 to 3 heteroatoms selected from the group of oxygen, nitrogen and sulfur, where the ring system is additionally substituted by N(R24)(R25) and may additionally be substituted by F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), CON(R27)(R28), hydroxy, hydroxy-(C₁-C₄)-alkyl, COO(R29), N(R30)CO(C₁-C₆)-alkyl or SO₂CH₃ ;
where
R26, R27, R28, R29, R30 are independently of one another H, (C₁-C₆)-alkyl;
and
R24, R25 are independently of one another H, (C₁-C₈)-alkyl, -(CH₂)ₒ-R36, CO-(C₁-C₈)-alkyl, or R24 and R25 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, Cl, (C₁-C₆)-alkyl, O-(C₁-C₈)-alkyl, oxo, CO(R44), CON(R45)(R46), hydroxy, N(R48)CO(C₁-C₆)-alkyl or N(R49)(R50);
o is 0, 1, 2, 3, 4;
R36 is OH, 5-10 membered mono- or bicyclic ring which may comprise one or more heteroatoms from the group of N, O and S, and the 5-10 membered ring may comprise further substituents such as F, Cl, Br, OH, CF₃, oxo, O-(C₁-C₆)-alkyl, (C₁-C₆)-alkyl, O-(C₀-C₂)-alkylene-aryl, (C₀-C₂)-alkylene-aryl and N(R55)(R56);
R44, R45, R46, R48, R49, R50 are independently of one another H, (C₁-C₆)-alkyl ;
and
R55, R56 are independently of one another H, (C₁-C₈)-alkyl.

9. A compound of the formula I as claimed in any of claims 1 to 3, in which the meanings are:
R1 (C₁-C₈)-alkyl, (C₁-C₄)-alkoxy-(C₁-C₄)-alkyl, a 3 to 10-membered mono- or bicyclic ring which may include 0 to 2 heteroatoms selected from the group of oxygen, nitrogen and sulfur, where the ring system may additionally be substituted by F, Cl, Br, CF₃, CN, (C₁-C₆)-alkyl, O-(C₁-C₈)-alkyl, oxo, CO(R3), CON(R4)(R5), N(R7)CO(C₁-C₆)-alkyl or SO₂CH₃;
R3, R4, R5, R7, R8, R9 independently of one another H, (C₁-C₈)-alkyl;
K a bond, OCH₂, CH₂O, (C(R10)(R11))_{z}, C≡C
z 1, 2;
R10, R11 independently of one another H, (C₁-C₈)-alkyl,
E 5-7 membered bivalent carbo- or heterocyclic ring structure having 0-2 heteroatoms from the group of N, O and S, which may optionally have substituents from the group of H, F, Cl, Br, OH, CF₃, NO₂, OCF₃, O-(C₁-C₆)-alkyl, (C₁-C₆)-alkyl, (C₂-C₆)-alkenyl, N(R12)(R13), SO₂-CH₃, CO(R20);
R12, R13 independently of one another H, (C₁-C₈)-alkyl;
R20 independently of one another H, (C₁-C₈)-alkyl;
X a bond;
D,G both CH;
R2 O-(C₁-C₆)-alkyl, (C₁-C₆)-alkyl;
n O or 1;
Q N(R24)(R25), a 3 to 8-membered monocyclic ring which may include 0 to 3 heteroatoms selected from the group of oxygen, nitrogen and sulfur, where the ring system may additionally be substituted by N(R24)(R25), F, Cl, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), N(R30)CO(C₁-C₆)-alkyl or SO₂CH₃;
R26, R30 (C₁-C₆)-alkyl;
A a group of the formula -(C(R31)(R32)ₘ-, in which 1 member may be replaced by an element from the group of O, N(R33);
m 0, 1, 3 or 4;
R31, R32, R33 H;
R24, R25 independently of one another H, (C₁-C₈)-alkyl, -(CH₂)ₒ-R36, CO(C₁-C₈)-alkyl, or R24 and R25 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, Cl, (C₁-C₆)-alkyl, O-(C₁-C₈)-alkyl, oxo, CO(R44), N(R48)CO(C₁-C₆)-alkyl or N(R49)(R50);
o 0, 1, 2, 3, 4;
R36 5-8 membered monocyclic ring which may comprise 0-2 heteroatoms from the group of N, O and S, and the 5-8 membered ring may comprise further substituents such as F, oxo, O-(C₁-C₆)-alkyl, (C₁-C₆)-alkyl, (C₀-C₂)-alkylene-aryl and N(R55)(R56);
R44,R48,R49,R50 independently of one another H, (C₁-C₆)-alkyl;
R55, R56 independently of one another H, (C₁-C)-alkyl;
and the physiologically tolerated salts thereof.

10. A compound of the formula I as claimed in any of claims 1 to 3, 5 or 6, wherein
A is a group of the formula -(C(R31)(R32))ₘ- in which 1 member may be replaced by an element from the group of O, N(R33);
where
m is 1, 3 or 4;
and
R31, R32, R33 are H;
and Q is N(R24)(R25);
where
R24, R25 independently of one another H, (C₁-C₈)-alkyl, -(CH₂)ₒ-R36, CO(C₁-C₈)-alkyl, or R24 and R25 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, (C₁-C₆)-alkyl, O-(C₁-C₈)-alkyl, oxo, CO(R44), N(R48)CO(C₁-C₆)-alkyl or N(R49)(R50);
o is 0, 1, 2, 3, 4;
R36 is OH, 5-8 membered monocyclic ring which may comprise 0-2 heteroatoms from the group of N, O and S, and the 5-8 membered ring may comprise further substituents such as F, oxo, O-(C₁-C₆)-alkyl, (C₁-C₆)-alkyl, (C₀-C₂)-alkylene-aryl and N(R55)(R56);
R44, R48, R49, R50 are independently of one another H, (C₁-C₆)-alkyl;
R55, R56 are independently of one another H, (C₁-C₈)-alkyl.

11. A compound of the formula I as claimed in any of claims 1 to 3, 5 or 7, in which
A is a group of the formula -(C(R31)(R32))ₘ-, in which 1 member may be replaced by an element from the group of O, N(R33);
where
m is 0 or 1 ;
and
R31, R32, R33 are H;
and
Q is a 5 to 7-membered monocyclic ring which comprises 1 or 2 nitrogen atoms, where the ring system may additionally be substituted by (C₁-C₆)-alkyl, (C₁-C₄)-alkoxy-(C₁-C₄)-alkyl, (C₀-C₈)-alkylene-aryl.

12. A compound of the formula I as claimed in any of claims 1 to 3, 5 or 8, wherein
A is a group of the formula -(C(R31)(R32))ₘ-, in which 1 member may be replaced by an element from the group of O, N(R33);
where
m is 0 or 1;
and
R31, R32, R33 are H;
Q is a 3 to 8-membered monocyclic ring which may include O to 1 heteroatoms selected from the group of oxygen, nitrogen and sulfur, where the ring system is additionally substituted by N(R24)(R25) and may additionally be substituted by F, Cl, Br, CF₃, NO₂, CN, (C₁-C₆)-alkyl, O-(C₁-C₈)-alkyl, (C₁-C₄)-alkoxy-(C₁-C₄)-alkyl, (C₀-C₈)-alkylene-aryl, oxo, CO(R26), CON(R27)(R28), hydroxy, hydroxy-(C₁-C₄)-alkyl, COO(R29), N(R30)CO(C₁-C₆)-alkyl or SO₂CH₃ ;
where
R26, R27, R28, R29, R30 are (C₁-C₆)-alkyl;
and
R24, R25 are independently of one another H, (C₁-C₈)-alkyl, -(CH₂)ₒ-R36, CO-(C₁C₈)-alkyl, or R24 and R25 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, (C₁-C₆)-alkyl, O-(C₁-C₈)-alkyl, oxo, CO(R44), N(R48)CO(C₁-C₆)-alkyl or N(R49)(R50);
o is 0, 1, 2, 3, 4;
R36 is a 5-8 membered monocyclic ring which may comprise 0-2 heteroatoms from the group of N, O and S, and the 5-8 membered ring may comprise further substituents such as F, oxo, O-(C₁-C₆)-alkyl, (C₁-C₆)-alkyl, (C₀-C₂)-alkylene-aryl and N(R55)(R56).

13. A process for preparing compounds as claimed in any of claims 1 to 12, whose A-Q group is linked via a nitrogen atom to the cyclohexylene ring, comprising the steps of
a) preparation of a compound of the formula II by reacting cyclohexadione with 1,2-ethylene glycol 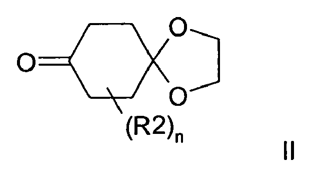
b) reaction of the compound of the formula II with 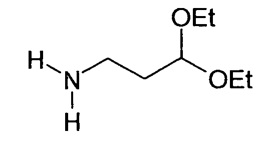 to give a compound of the formula III 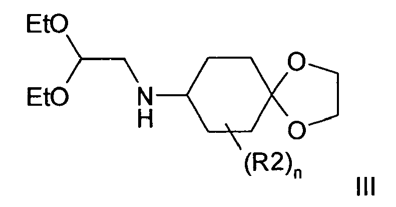
c) coupling of the compound of the formula III with a primary amine of the formula 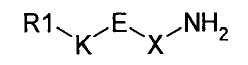 and subsequent deprotection of the acetal, with simultaneous cyclization resulting in a compound of the formula IV 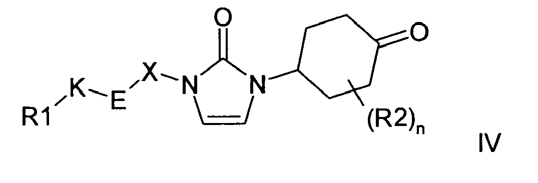
d) reaction with a secondary amine, resulting in compounds of the formula I whose A-Q group is linked via a nitrogen atom to the cyclohexylene ring

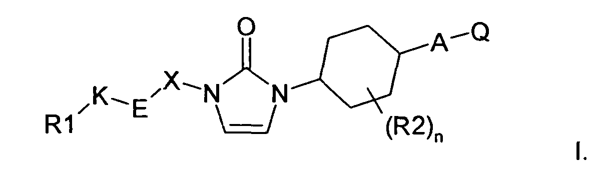

14. A medicament comprising one or more of the compounds as claimed in any of claims 1 to 12.

15. A medicament comprising one or more of the compounds as claimed in any of claims 1 to 12 and one or more anorectic active ingredients.

16. A compound of the formula I as claimed in any of claims 1 to 12 for use as medicament for the prophylaxis or treatment of obesity.

17. A compound of the formula I as claimed in any of claims 1 to 12 for use as medicament for the prophylaxis or treatment of type II diabetes.

18. A compound of the formula I as claimed in any of claims 1 to 12 in combination with at least one further anorectic active ingredient for use as medicament for the prophylaxis or treatment of obesity.

19. A compound of the formula I as claimed in any of claims 1 to 12 in combination with at least one further anorectic active ingredient for use as medicament for the prophylaxis or treatment of type II diabetes.

20. A process for producing a medicament comprising one or more of the compounds of the formula I as claimed in any of claims 1 to 12, which comprises mixing the active ingredient with a pharmaceutically acceptable carrier and converting this mixture into a form suitable for administration.

21. The use of the compounds of the formula I as claimed in any of claims 1 to 12 for producing a medicament for weight reduction in mammals.

22. The use of the compounds of the formula I as claimed in any of claims 1 to 12 for producing a medicament for the prophylaxis or treatment of obesity.

23. The use of the compounds of the formula I as claimed in any of claims 1 to 12 for producing a medicament for the prophylaxis or treatment of type II diabetes.

## Revendications

1. Composés de formule I 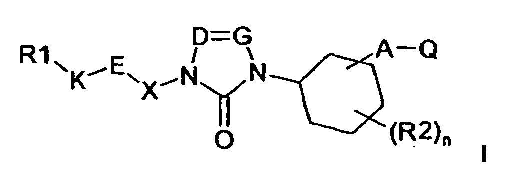 dans laquelle
R1 signifie H, (C₁-C₈)-alkyle, (C₁-C₄) -alcoxy- (C₁-C₄) -alkyle, (C₃-C₈) -alcényle, (C₃-C₈)-alcynyle, un cycle monocyclique, bicyclique ou spirocyclique de 3 à 10 chaînons, qui peut comporter 0 à 4 hétéroatomes, choisis dans le groupe formé par l'oxygène, l'azote et le soufre, le système cyclique pouvant en outre être substitué par F, Cl, Br, CF₃, NO₂, CN, (C₁-C₆)-alkyle, O-(C₁-C₈)-alkyle, S-(C₁-C₈)-alkyle, (C₁-C₄) -alcoxy- (C₁-C₄) -alkyle, (C₀-C₈) -alkylénaryle, oxo, CO(R3), CON(R4)(R5), hydroxy, hydroxy- (C₁-C₄) -alkyle, COO (R6), N (R7) CO (C₁-C₆)-alkyle, N (R8) (R9) ou SO₂CH₃ ;
R3, R4, R5, R6, R7, R8, R9 signifient, indépendamment l'un de l'autre H, (C₁-C₆)-alkyle ;
K signifie un groupe de formule -(CR10R11)_{z}-, où un ou plusieurs groupes -(CR10R11)- peuvent être remplacés par Z, une liaison, C=C, C=C ;
Z 0, CO, N(R59), S, SO, SO₂ ;
R10, R11 signifient, indépendamment l'un de l'autre H, (C₁-C₈)-alkyle, hydroxy-(C₁-C₄)-alkyle, hydroxy, (C₁-C₄)-alcoxy-(C₁-C₄)-alkyle, où R10 et R11 dans les z groupes peuvent présenter à chaque fois des significations identiques ou différentes ;
z vaut 1, 2, 3, 4, 5, 6 ;
R59 signifie H, (C₁-C₈)-alkyle ;
E signifie une structure carbocyclique ou hétérocyclique divalente de 3-14 chaînons comprenant 0-4 hétéroatomes du groupe N, O et S, qui peut éventuellement porter des substituants du groupe formé par H, F, Cl, Br, I, OH, CF₃, NO₂, CN, OCF₃, oxo, O-(C₁-C₆)-alkyle, O-(C₁-C₄)-alcoxy-(C₁-C₄)-alkyle, S-(C₁-C₆)-alkyle, (C₁-C₆)-alkyle, (C₂-C₆)-alcényle, (C₃-C₈)-cycloalkyle, O-(C₃-C₈)-cycloalkyle, (C₃-C₈)-cycloalcényle, O-(C₃-C₈)-cycloalcényle, (C₂-C₆) -alcynyle, (C₀-C₈) -alkylénaryle, O- (C₀-C₈) -alkylénaryle, S-aryle, N(R12)(R13), SO₂-CH₃, COOH, COO-(C₁-C₆)-alkyle, CON(R14)(R15), N(R16)CO(R17), N(R18)SO₂(R19), CO(R20) et qui peut être monocyclique ou bicyclique ;
R12, R13, R14, R15, R16, R18 signifient, indépendamment l'un de l'autre H, (C₁-C₈)-alkyle ;
ou
R12 et R13, R14 et R15 signifient, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe N-(C₁-C₆)-alkyle, oxygène et soufre ;
R17, R19, R20 signifient, indépendamment l'un de l'autre H, (C₁-C₈)-alkyle, aryle ;
X signifie une liaison, un groupe de formule -(CR21R22)_{y}-, où un ou plusieurs groupes -(CR21R22)- peuvent être remplacés par Y ;
Y signifie O, CO, N(R23), S, SO, SO₂ ;
R21, R22 signifient, indépendamment l'un de l'autre H, (C₁-C₄)-alkyle, où R21 et R22 dans les y groupes peuvent à chaque fois présenter des significations identiques ou différentes ;
y vaut 1, 2, 3, 4, 5, 6 ;
R23 signifie H, (C₁-C₈)-alkyle ;
D, G signifie CH ou N ;
R2 signifie OH, O-(C₁-C₆)-alkyle, O-(C₁-C₄)-alcoxy- (C₁-C₄)-alkyle, (C₁-C₆)-alkyle ;
n vaut 0, 1, 2, 3, 4, ;
Q signifie N(R24)(R25), un cycle de 3 à 8 chaînons qui peut comporter 0 à 3 hétéroatomes, choisis dans le groupe formé par l'oxygène, l'azote et le soufre, le système cyclique pouvant en outre être substitué par (C₀-C₄)-alkylène-N(R24)(R25), F, Cl, Br, CF₃, NO₂, CN, (C₁-C₆)-alkyle, O(C₁-C₈)-alkyle, (C₁-C₄)-alcoxy-(C₁-C₄)-alkyle, (C₀-C₈)-alkylénaryle, oxo, CO(R26), CON(R27)(R28), hydroxy, hydroxy- (C₁-C₄) -alkyle, COO (R29), N(R30) CO (C₁-C₆)-alkyle ou SO₂CH₃ ;
R26, R27, R28, R29, R30 signifient, indépendamment l'un de l'autre H, (C₁-C₆)-alkyle ;
A signifie un groupe de formule -(C(R31)(R32))ₘ-, dans laquelle 0-2 membres peuvent être remplacés par un élément du groupe O, S, N(R33), CO, SO₂ ;
m vaut 0, 1, 2, 3, 4, 5 ;
R31, R32, R33 signifient, indépendamment l'un de l'autre H, (C₁-C₆)-alkyle, aryle ;
R24, R25 signifient indépendamment l'un de l'autre H, (C₁-C₈) -alkyle, - (CR34R35)ₒ-R36, (C₁-C₄) -alcoxy- (C₁-C₄) -alkyle, (C₃-C₈) -alcényle, (C₃-C₈)-alcynyle, CO (C₁-C₈) -alkyle, -CO- (CH₂)ₒ-R36, CO(C(R37) (R38))_{q}N(R39) (R40),
CO(C(R41)(R42))ₛO(R43) ; ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 4 hétéroatomes supplémentaires, choisis dans le groupe formé par l'oxygène, l'azote et le soufre, le système hétérocyclique pouvant en outre être substitué par F, Cl, Br, CF₃, NO₂, CN, (C₁-C₆)-alkyle, O-(C₁-C₈)-alkyle, (C₁-C₄)-alcoxy-(C₁-C₄)-alkyle, hydroxy- (C₁-C₄) -alkyle, (C₀-C₈) -alkylénaryle, oxo, CO(R44), CON(R45)(R46), hydroxy, COO(R47), N(R48)CO(C₁-C₆)-alkyle, N(R49)(R50) ou SO₂CH₃;
o vaut 0, 1, 2, 3, 4, 5, 6 ;
q, s valent, indépendamment l'un de l'autre 0, 1, 2, 3, 4 ;
R34, R35 signifient, indépendamment l'un de l'autre H, (C₁-C₈)-alkyle, hydroxy-(C₁-C₄)alkyle, OH, (C₁-C₄)-alcoxy-(C₁-C₄)-alkyle ;
R37, R38, R39, R40, R41, R42, R43, R44, R45, R46, R47, R48, R49, R50 signifient, indépendamment l'un de l'autre H, (C₁-C₆)-alkyle ;
R39 et R40, R45 et R46, R49 et R50 signifient indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe N-(C₁-C₆)-alkyle, oxygène et soufre.
R36 signifie OH, O-(C₁-C₆)-alkyle, O- (C₀-C₈) -alkylénaryle, CON (R51) (R52), N(R53)(R54), un cycle de 3-12 chaînons, monocyclique, bicyclique ou spirocyclique, qui peut contenir un ou plusieurs hétéroatomes du groupe formé par N, O et S et le cycle de 3-12 chaînons peut contenir d'autres substituants tels que F, Cl, Br, I, OH, CF₃, NO₂, CN, OCF₃, oxo, O-(C₁-C₆)-alkyle, (C₁-C₄) -alcoxy- (C₁-C₄) -alkyle, S- (C₁-C₆) -alkyle, (C₁-C₆)-alkyle, (C₂-C₆)-alcényle, (C₃-C₈)-cycloalkyle, O-(C₃-C₈)-cycloalkyle, (C₃-C₈)-cycloalcényle, O-(C₃-C₈)-cycloalcényle, (C₂-C₆)-alcynyle, 0-(C₀-C₈)-alkylénaryle, (C₀-C₈)-alkylénaryle, N (R55) (R56), CO(C₁-C₆)-alkyle, COO(R57) et S(O)ᵤ(R58) ;
u vaut 0, 1, 2, ;
R51, R52 signifient, indépendamment l'un de l'autre H, (C₁-C₈)-alkyle, (C₂-C₆) alcényle, (C₀-C₈)alkylénaryle ;
R53, R54 signifient, indépendamment l'un de l'autre H, (C₁-C₆)-alkyle ;
R55, R56 signifient, indépendamment l'un de l'autre H, (C₁-C₈)-alkyle ;
R57 signifie H, (C₁-C₈)-alkyle, (C₂-C₆)-alcényle, (C₀-C₈)-alkylénaryle ;
R58 signifie (C₁-C₆)-alkyle, un système cyclique aromatique de 5-10 chaînons, qui peut contenir 0-2 autres hétéroatomes du groupe formé par l'azote, l'oxygène et le soufre et qui peut être substitué par F, Cl, Br, CF₃, NO₂, CN, (C₁-C₈)-alkyle, O-(C₁-C₈)-alkyle ;
ainsi que leurs sels physiologiquement acceptables.

2. Composés de formule I selon la revendication 1, dans laquelle :
R1 signifie (C₁-C₈)-alkyle, (C₁-C₄) -alcoxy- (C₁-C₄) -alkyle, un cycle de 3 à 10 chaînons, monocyclique, bicyclique ou spirocyclique, qui peut comporter 0 à 3 hétéroatomes, choisis dans le groupe formé par l'oxygène, l'azote et le soufre, le système cyclique pouvant en outre être substitué par F, Cl, Br, CF₃, CN, (C₁-C₆)-alkyle, O-(C₁-C₈)-alkyle, (C₀-C₂)-alkylénaryle, oxo, CO (R3), CON(R4)(R5), hydroxy, N (R7) CO (C₁-C₆) -alkyle, N (R8) (R9) ou SO₂CH₃;
R3, R4, R5, R7, R8, R9 signifient, indépendamment l'un de l'autre H, (C₁-C₈)-alkyle;
K signifie une liaison, OCH₂, CH₂O, (C(R10) (R11))_{z}, C≡C ;
z vaut 1, 2, 3, 4 ;
R10, R11 signifient, indépendamment l'un de l'autre H, (C₁-C₈)-alkyle;
E signifie une structure carbocyclique ou hétérocyclique divalente de 3-8 chaînons comprenant 0-4 hétéroatomes du groupe N, O et S, qui peut éventuellement porter des substituants du groupe formé par H, F, Cl, Br, OH, CF₃, N0₂, CN, OCF₃, O-(C₁-C₆)-alkyle, O-(C₁-C₄)-alcoxy-(C₁-C₄)-alkyle, S-(C₁-C₆)-alkyle, (C₁-C₆)-alkyle, (C₂-C₆)-alcényle, N(R12) (R13), SO₂-CH₃, N(R16) CO (R17), N(R18)SO₂(R19), CO (R20) et qui peut être monocyclique ou bicyclique ;
R12, R13, R16, R18 signifient, indépendamment l'un de l'autre H, (C₁-C₈)-alkyle ;
R17, R19, R20 signifient, indépendamment l'un de l'autre H, (C₁-C₈)-alkyle, aryle ;
X signifie une liaison, -CH₂-CH₂- ;
D, G soit D représente N et G représente CH ou D représente CH et G représente N ou D et G représentent simultanément CH ;
R2 signifie OH, O-(C₁-C₆)-alkyle, O-(C₁-C₄)-alcoxy-(C₁-C₄)-alkyle, (C₁-C₆)-alkyle ;
n vaut 0, 1, 2 ;
Q signifie N(R24)(R25), un cycle monocyclique de 3 à 8 chaînons qui peut comporter 0 à 3 hétéroatomes, choisis dans le groupe formé par l'oxygène, l'azote et le soufre, le système cyclique pouvant en outre être substitué par N(R24)(R25), F, Cl, Br, CF₃, NO₂, CN, (C₁-C₆) -alkyle, O-(C₁-C₈)-alkyle, (C₁-C₄) -alcoxy- (C₁-C₄) -alkyle, (C₀-C₈) -alkylénaryle, oxo, CO(R26), CON(R27)(R28), hydroxy, hydroxy-(C₁-C₄)-alkyle, COO (R29), N(R30)CO(C₁-C₆)-alkyle ou SO₂CH₃ ;
R26, R27, R28, R29, R30 signifient, indépendamment l'un de l'autre H, (C₁-C₆)-alkyle ;
A signifie -(C(R31)(R32))ₘ-, où 0-2 membres peuvent être remplacés par un élément du groupe O, N(R33), CO ;
m vaut 0, 1, 2, 3, 4 ;
R31, R32, R33 signifient, indépendamment l'un de l'autre H, (C₁-C₆)-alkyle, aryle ;
R24, R25 signifient indépendamment l'un de l'autre H, (C₁-C₈)-alkyle, - (CH₂)ₒ-R36, CO (C₁-C₈)-alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par F, Cl, (C₁-C₆)-alkyle, O-(C₁-C₈)-alkyle, oxo, CO(R44), CON (R45) (R46), hydroxy, N(R48)CO(C₁-C₆)-alkyle ou N(R49)(R50) ;
o vaut 0, 1, 2, 3, 4 ;
R36 signifie OH, un cycle monocyclique ou bicyclique de 5-10 chaînons, qui peut contenir un ou plusieurs hétéroatomes du groupe N, 0 et S et le cycle de 5-10 chaînons peut contenir d'autres substituants tels que F, Cl, Br, OH, CF₃, oxo, O-(C₁-C₆)-alkyle, (C₁-C₆)-alkyle, O-(C₀-C₂)-alkylénaryle, (C₀-C₂)-alkylénaryle et N (R55) (R56) ;
R44, R45, R46, R48, R49, R50 signifient, indépendamment l'un de l'autre H, (C₁-C₆)-alkyle ;
R55, R56 signifient, indépendamment l'un de l'autre H, (C₁-C₈)-alkyle ;
ainsi que leurs sels physiologiquement acceptables.

3. Composés de formule I selon la revendication 1 ou 2, où
R1 signifie (C₁-C₈)-alkyle, (C₁-C₄)-alcoxy-(C₁-C₄)-alkyle, un cycle de 3 à 10 chaînons, monocyclique ou bicyclique, qui peut comporter 0 à 2 hétéroatomes, choisis dans le groupe formé par l'oxygène, l'azote et le soufre, le système cyclique pouvant en outre être substitué par F, Cl, Br, CF₃, CN, (C₁-C₆) -alkyle, O-(C₁-C₈)-alkyle, oxo, CO(R3), CON(R4)(R5), N(R7)CO(C₁-C₆)-alkyle ou SO₂CH₃ ;
R3, R4, R5, R7, R8, R9 signifient, indépendamment l'un de l'autre H, (C₁-C₈)-alkyle ;
K signifie une liaison, OCH₂, CH₂O, (C(R10)(R11))_{z}, C≡C ;
z vaut 1, 2 ;
R10, R11 signifient, indépendamment l'un de l'autre H, (C₁-C₈)-alkyle ;
E signifie une structure carbocyclique ou hétérocyclique divalente de 5-7 chaînons comprenant 0-2 hétéroatomes du groupe N, O et S, qui peut éventuellement porter des substituants du groupe formé par H, F, Cl, Br, OH, CF₃, NO₂, OCF₃, O-(C₁-C₆)-alkyle, (C₁-C₆)-alkyle, (C₂-C₆)-alcényle, N(R12)(R13), SO₂-CH₃, CO (R20) ;
R12, R13 signifient, indépendamment l'un de l'autre H, (C₁-C₈)-alkyle ;
R20 signifie, indépendamment l'un de l'autre H, (C₁-C₈)-alkyle ;
X signifie une liaison ;
D, G signifient simultanément CH ;
R2 signifie OH, O- (C₁-C₆) -alkyle, 0- (C₁-C₄) -alcoxy- (C₁-C₄) -alkyle, (C₁-C₆) -alkyle ;
n vaut 0 ou 1 ;
Q signifie N(R24)(R25), un cycle monocyclique de 3 à 8 chaînons qui peut comporter 0 à 3 hétéroatomes, choisis dans le groupe formé par l'oxygène, l'azote et le soufre, le système cyclique pouvant en outre être substitué par N(R24)(R25), F, Cl, Br, CF₃, NO₂, CN, (C₁-C₆)-alkyle, O-(C₁-C₈)-alkyle, (C₁-C₄) -alcoxy- (C₁-C₄) -alkyle, (C₀-C₈) -alkylénaryle, oxo, CO(R26), CON(R27)(R28), hydroxy, hydroxy-(C₁-C₄)-alkyle, COO(R29), N (R30) CO (C₁-C₆) -alkyle ou SO₂CH₃ ;
R26, R27, R28, R29, R30 signifient (C₁-C₆) -alkyle ;
A signifie un groupe de formule -(C(R31)(R32))ₘ-, dans laquelle 1 membre peut être remplacé par un élément du groupe O, N(R33) ;
m vaut 0, 1, 3 ou 4 ;
R31, R32, R33 signifient H ;
R24, R25 signifient indépendamment l'un de l'autre H, (C₁-C₈)-alkyle, -(CH₂)ₒ-R36, CO (C₁-C₈) -alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par F, Cl, (C₁-C₆)-alkyle, O-(C₁-C₈)-alkyle, oxo, CO(R44), CON (R45) (R46), hydroxy, N(R48)CO(C₁-C₆)-alkyle ou N(R49)(R50) ;
o vaut 0, 1, 2, 3, 4 ;
R36 signifie OH, un cycle monocyclique ou bicyclique de 5-10 chaînons, qui peut contenir un ou plusieurs hétéroatomes du groupe N, O et S et le cycle de 5-10 chaînons peut contenir d'autres substituants tels que F, Cl, Br, OH, CF₃, oxo, O-(C₁-C₆)-alkyle, (C₁-C₆)-alkyle, O-(C₀-C₂)-alkylénaryle, (C₀-C₂)-alkylénaryle et N (R55) (R56) ;
R44, R45, R46, R48, R49, R50 signifient, indépendamment l'un de l'autre H, (C₁-C₆)-alkyle ;
R55, R56 signifient, indépendamment l'un de l'autre H, (C₁-C₈)-alkyle ;
ainsi que leurs sels physiologiquement acceptables.

4. Composés de formule I selon la revendication 1, où les radicaux R1 et R2, l'indice n et les groupes K, E, X, D=G, Q et A présentent les significations suivantes
R1 signifie H, (C₁-C₈)-alkyle, (C₁-C₄)-alcoxy-(C₁-C₄)-alkyle, un cycle monocyclique de 3 à 8 chaînons, qui peut comporter 0 à 3 hétéroatomes, choisis dans le groupe formé par l'oxygène, l'azote et le soufre, le système cyclique pouvant en outre être substitué par F, Cl, Br, (C₁-C₆) alkyle, O-(C₁-C₈)-alkyle, (C₁-C₄)-alcoxy-(C₁-C₄)-alkyle,
K signifie O, une liaison, C≡C, CO, OCH₂, OCH₂CH₂ ;
E signifie une structure carbocyclique ou hétérocyclique monocyclique divalente de 5-6 chaînons comprenant 0-2 hétéroatomes du groupe N, O et S, qui peut éventuellement porter des substituants du groupe formé par H, F, Cl, Br, oxo, O- (C₁-C₆) -alkyle, O-(C₁-C₄)-alcoxy-(C₁-C₄)-alkyle, (C₁-C₆)-alkyle ;
X signifie une liaison, CH₂CH₂, CH₂CH₂CH₂, OCH₂CH₂ ;
D=G signifie CH=CH, CH=N, N=CH ;
R2 signifie OH, O-(C₁-C₆)-alkyle, (C₁-C₆)-alkyle ;
n vaut 0, 1, 2 ;
Q signifie un groupe de formule N(R24)(R25), un cycle azoté de 4 à 8 chaînons qui peut comporter outre l'atome d'azote encore 0-2 hétéroatomes, choisis dans le groupe formé par l'oxygène, l'azote et le soufre, le système cyclique pouvant en outre être substitué par N(R24)(R25), F, (C₁-C₆)-alkyle, 0 (C₁-C₈)-alkyle, (C₁-C₄) -alcoxy- (C₁-C₄) -alkyle, (C₀-C₈) -alkylénaryle, oxo, CO(R26), N(R30)CO(C₁-C₆)-alkyle ou SO₂CH₃ ;
R24, R25 signifient indépendamment l'un de l'autre H, (C₁-C₈)-alkyle, (C₁-C₄) -alcoxy- (C₁-C₄) -alkyle, CO (C₁-C₈) -alkyle ou R24 et R25 forment ensemble avec l'atome d'azote auquel ils sont liés un cycle de 4 à 8 chaînons, monocyclique qui peut contenir, outre l'atome d'azote, 0 à 1 hétéroatome supplémentaire, choisi dans le groupe formé par l'oxygène, l'azote et le soufre, le système cyclique hétérocyclique pouvant en outre être substitué par (C₁-C₆)-alkyle, N(R48)CO(C₁-C₆)-alkyle ou N (R49) (R50) ;
R48, R49, R50 signifient, indépendamment l'un de l'autre H, (C₁-C₆)-alkyle ;
R49 et R50 signifient éventuellement avec l'atome d'azote auquel ils sont liés, un cycle de 5-6 chaînons, qui peut encore contenir, outre l'atome d'azote, 0-1 autre hétéroatome du groupe N-(C₁-C₆)-alkyle, oxygène et soufre ;
R26, R30 signifient, indépendamment l'un de l'autre H, (C₁-C₆) -alkyle ;
A signifie un groupe de formule -(C(R31)(R32))ₘ-, dans laquelle 1 membre peut être remplacé par un élément du groupe O, N(R33) ;
m vaut 0, 1, 3 ;
R31, R32, R33 signifient, indépendamment l'un de l'autre H, (C₁-C₆)-alkyle, aryle.

5. Composés de formule I selon l'une quelconque des revendications 1 à 4, **caractérisés en ce que** D et G signifient à chaque fois CH.

6. Composés de formule I selon l'une quelconque des revendications 1 à 3 ou 5, **caractérisés en ce que**
A signifie un groupe de formule -(C(R31) (R32))ₘ-, dans laquelle 0-2 membres peuvent être remplacés par un élément du groupe O, N(R33), CO ;
où
m vaut 1, 2, 3, 4 ;
et
R31, R32, R33 signifient, indépendamment l'un de l'autre H, (C₁-C₆)-alkyle, aryle ;
et
Q signifie N(R24) (R25) ;
où
R24, R25 signifient indépendamment l'un de l'autre H, (C₁-C₈)-alkyle, -(CH₂)ₒ-R36, CO(C₁-C₈)-alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par F, Cl, (C₁-C₆)-alkyle, O-(C₁-C₈)-alkyle, oxo, CO(R44), CON (R45) (R46), hydroxy, N(R48)CO(C₁-C₆)-alkyle ou N(R49)(R50) ;
o vaut 0, 1, 2, 3, 4, ;
R36 signifie OH, un cycle monocyclique ou bicyclique de 5-10 chaînons, qui peut contenir un ou plusieurs hétéroatomes du groupe N, O et S et le cycle de 5-10 chaînons peut contenir d'autres substituants tels que F, Cl, Br, OH, CF₃, oxo, O-(C₁-C₆)-alkyle, (C₁-C₆)-alkyle, O-(C₀-C₂)-alkylénaryle, (C₀-C₂)-alkylénaryle et N(R55)(R56) ;
R44, R45, R46, R48, R49, R50 signifient, indépendamment l'un de l'autre H, (C₁-C₆)-alkyle ;
et
R55, R56 signifient, indépendamment l'un de l'autre H, (C₁-C₈)-alkyle.

7. Composés de formule I selon l'une quelconque des revendications 1 à 3 ou 5, **caractérisés en ce que**
A signifie un groupe de formule -(C(R31)(R32))ₘ-, dans laquelle 0-2 membres peuvent être remplacés par un élément du groupe O, N(R33), CO ;
où
m vaut 0, 1, 2, 3, 4 ;
et
R31, R32, R33 signifient, indépendamment l'un de l'autre H, (C₁-C₆)-alkyle, aryle ;
et
Q signifie un cycle monocyclique de 3 à 8 chaînons qui peut comporter 0 à 3 hétéroatomes, choisis dans le groupe formé par l'oxygène, l'azote et le soufre, le système cyclique pouvant en outre être substitué par F, Cl, Br, CF₃, NO₂, CN, (C₁-C₆)-alkyle, O-(C₁-C₈)-alkyle, (C₁-C₄) -alcoxy- (C₁-C₄) -alkyle, (C₀-C₈) -alkylénaryle, oxo, CO (R26), CON (R27) (R28), hydroxy, hydroxy-(C₁-C₄)-alkyle, COO (R29), N(R30)CO(C₁-C₆)-alkyle ou SO₂CH₃ ;
où
R26, R27, R28, R29, R30 signifient, indépendamment l'un de l'autre H, (C₁-C₆)-alkyle.

8. Composés selon l'une quelconque des revendications 1 à 3 ou 5, **caractérisés en ce que**
A signifie un groupe de formule -(C(R31)(R32))ₘ-, dans laquelle 0-2 membres peuvent être remplacés par un élément du groupe O, N(R33), CO ;
où
m vaut 0, 1, 2, 3, 4 ;
et
R31, R32, R33 signifient, indépendamment l'un de l'autre H, (C₁-C₆)-alkyle, aryle ;
et
Q signifie un cycle monocyclique de 3 à 8 chaînons qui peut comporter 0 à 3 hétéroatomes, choisis dans le groupe formé par l'oxygène, l'azote et le soufre, le système cyclique étant en outre substitué par N(R24)(R25) et pouvant en outre être substitué par F, Cl, Br, CF₃, NO₂, CN, (C₁-C₆)-alkyle, O-(C₁-C₈)-alkyle, (C₁-C₄)-alcoxy-(C₁-C₄)-alkyle, (C₀-C₈)-alkylénaryle, oxo, CO(R26), CON(R27)(R28), hydroxy, hydroxy- (C₁-C₄) -alkyle, COO (R29), N(R30)CO(C₁-C₆)-alkyle ou SO₂CH₃ ;
où
R26, R27, R28, R29, R30 signifient, indépendamment l'un de l'autre H, (C₁-C₆)-alkyle.
et
R24, R25 signifient indépendamment l'un de l'autre H, (C₁-C₈)-alkyle, -(CH₂)ₒ-R36, CO(C₁-C₈)-alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système cyclique hétérocyclique pouvant en outre être substitué par F, Cl, (C₁-C₆)-alkyle, O-(C₁-C₈)-alkyle, oxo, CO(R44), CON (R45) (R46), hydroxy, N (R48) CO (C₁-C₆)-alkyle ou N (R49) (R50) ;
o vaut 0, 1, 2, 3, 4 ;
R36 signifie OH, un cycle monocyclique ou bicyclique de 5-10 chaînons, qui peut contenir un ou plusieurs hétéroatomes du groupe N, 0 et S et le cycle de 5-10 chaînons peut contenir d'autres substituants tels que F, Cl, Br, OH, CF₃, oxo, O-(C₁-C₆)-alkyle, (C₁-C₆)-alkyle, O-(C₀-C₂)-alkylénaryle, (C₀-C₂)-alkylénaryle et N (R55) (R56) ;
R44, R45, R46, R48, R49, R50 signifient, indépendamment l'un de l'autre H, (C₁-C₆) -alkyle ;
et
R55, R56 signifient, indépendamment l'un de l'autre H, (C₁-C₈)-alkyle.

9. Composés de formule I selon l'une quelconque des revendications 1 à 3, où
R1 signifie (C₁-C₈) -alkyle, (C₁-C₄)-alcoxy-(C₁-C₄)-alkyle, un cycle de 3 à 10 chaînons, monocyclique ou bicyclique, qui peut comporter 0 à 2 hétéroatomes, choisis dans le groupe formé par l'oxygène, l'azote et le soufre, le système cyclique pouvant en outre être substitué par F, Cl, Br, CF₃, CN, (C₁-C₆)-alkyle, O-(C₁-C₈) -alkyle, oxo, CO(R3), CON(R4)(R5), N(R7)CO(C₁-C₆)-alkyle ou SO₂CH₃ ;
R3, R4, R5, R7, R8, R9 signifient, indépendamment l'un de l'autre H, (C₁-C₈)-alkyle ;
K signifie une liaison, OCH₂, CH₂O, (C(R10)(R11))_{z}, C=C ;
z vaut 1, 2 ;
R10, R11 signifient, indépendamment l'un de l'autre H, (C₁-C₈)-alkyle ;
E signifie une structure carbocyclique ou hétérocyclique divalente de 5-7 chaînons comprenant 0-2 hétéroatomes du groupe N, O et S, qui peut éventuellement porter des substituants du groupe formé par H, F, Cl, Br, OH, CF₃, NO₂, OCF₃, O- (C₁-C₆) -alkyle, (C₁-C₆) -alkyle, (C₂-C₆) -alcényle, N(R12)(R13), SO₂-CH₃, CO(R20);
R12, R13 signifient, indépendamment l'un de l'autre H, (C₁-C₈)-alkyle ;
R20 signifie, indépendamment l'un de l'autre H, (C₁-C₈)-alkyle ;
X signifie une liaison ;
D, G signifient simultanément CH ;
R2 signifie O-(C₁-C₆)-alkyle, (C₁-C₆)-alkyle ;
n vaut 0 ou 1 ;
Q signifie N(R24)(R25), un cycle monocyclique de 3 à 8 chaînons qui peut comporter 0 à 3 hétéroatomes, choisis dans le groupe formé par l'oxygène, l'azote et le soufre, le système cyclique pouvant en outre être substitué par N(R24)(R25), F, Cl, (C₁-C₆)-alkyle, O-(C₁-C₈)-alkyle, (C₁-C₄) -alcoxy- (C₁-C₄) -alkyle, (C₀-C₈) -alkylénaryle, oxo, CO (R26), N (R30) CO (C₁-C₆) -alkyle ou SO₂CH₃ ;
R26, R30 signifient (C₁-C₆) -alkyle ;
A signifie un groupe de formule -(C(R31)(R32))ₘ-, dans laquelle 1 membre peut être remplacé par un élément du groupe O, N(R33) ;
m vaut 0, 1, 3 ou 4 ;
R31, R32, R33 signifient H ;
R24, R25 signifient indépendamment l'un de l'autre H, (C₁-C₈)-alkyle, -(CH₂)ₒ-R36, CO(C₁-C₈)-alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par F, Cl, (C₁-C₆)-alkyle, O-(C₁-C₈)-alkyle, oxo, CO(R44), N (R48) CO (C₁-C₆) -alkyle ou N(R49)(R50) ;
o vaut 0, 1, 2, 3, 4 ;
R36 signifie un cycle monocyclique de 5-8 chaînons, qui peut contenir 0-2 hétéroatomes du groupe formé par N, O et S et le cycle de 5-8 chaînons pouvant contenir d'autres substituants tels que F, oxo, O-(C₁-C₆)-alkyle, (C₁-C₆)-alkyle, (C₀-C₂)-alkylénaryle et N (R55) (R56) ;
R44, R48, R49, R50 signifient, indépendamment l'un de l'autre H, (C₁-C₆)-alkyle ;
R55, R56 signifient, indépendamment l'un de l'autre H, (C₁-C₈)-alkyle ;
ainsi que leurs sels physiologiquement acceptables.

10. Composés de formule I selon l'une quelconque des revendications 1 à 3, 5 ou 6, **caractérisés en ce que**
A signifie un groupe de formule -(C(R31)(R32))ₘ-, dans laquelle 1 membre peut être remplacé par un élément du groupe O, N(R33) ;
où
m vaut 1, 3 ou 4 ;
et
R31, R32, R33 signifient H ;
et Q signifie N(R24)(R25) ;
où
R24, R25 signifient indépendamment l'un de l'autre H, (C₁-C₈) -alkyle, - (CH₂)ₒ-R36, CO(C₁-C₈)-alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par F, (C₁-C₆)-alkyle, O-(C₁-C₈)-alkyle, oxo, CO(R44), N(R48)CO(C₁-C₆)-alkyle ou N (R49) (R50) ;
o vaut 0, 1, 2, 3, 4 ;
R36 signifie OH, un cycle monocyclique de 5-8 chaînons, qui peut contenir 0-2 hétéroatomes du groupe formé par N, O et S et le cycle de 5-8 chaînons pouvant contenir d'autres substituants tels que F, oxo, O-(C₁-C₆)-alkyle, (C₁-C₆)-alkyle, (C₀-C₂)-alkylénaryle et N (R55) (R56) ;
R44, R48, R49, R50 signifient, indépendamment l'un de l'autre H, (C₁-C₆)-alkyle ;
R55, R56 signifient, indépendamment l'un de l'autre H, (C₁-C₈)-alkyle.

11. Composés de formule I selon l'une quelconque des revendications 1 à 3, 5 ou 7, où
A signifie un groupe de formule -(C(R31)(R32))ₘ-, dans laquelle 1 membre peut être remplacé par un élément du groupe O, N(R33) ;
où
m vaut 0 ou 1 ;
et
R31, R32, R33 signifient H ;
et
Q signifie un cycle monocyclique de 5 à 7 chaînons, qui contient 1 ou 2 atomes d'azote, le système cyclique pouvant en outre être substitué par (C₁-C₆)-alkyle, (C₁-C₄) -alcoxy-(C₁-C₄) -alkyle, (C₀-C₈)-alkylénaryle.

12. Composés de formule I selon l'une quelconque des revendications 1 à 3, 5 ou 8, **caractérisés en ce que**
A signifie un groupe de formule -(C(R31)(R32))ₘ-, dans laquelle 1 membre peut être remplacé par un élément du groupe O, N(R33) ;
où
m vaut 0 ou 1 ;
et
R31, R32, R33 signifient H ;
Q signifie un cycle monocyclique de 3 à 8 chaînons qui peut comporter 0 à 1 hétéroatome, choisi dans le groupe formé par l'oxygène, l'azote et le soufre, le système cyclique étant en outre substitué par N(R24)(R25) et pouvant en outre être substitué par F, Cl, Br, CF₃, N0₂, CN, (C₁-C₆)-alkyle, O-(C₁-C₈)-alkyle, (C₁-C₄) -alcoxy- (C₁-C₄) -alkyle, (C₀-C₈) -alkylénaryle, oxo, CO(R26), CON(R27)(R28), hydroxy, hydroxy-(C₁-C₄)-alkyle, COO(R29), N(R30)CO(C₁-C₆)-alkyle ou SO₂CH₃ ;
où
R26, R27, R28, R29, R30 signifient (C₁-C₆)-alkyle ;
et
R24, R25 signifient indépendamment l'un de l'autre H, (C₁-C₈)-alkyle, -(CH₂)ₒ-R36, CO(C₁-C₈)-alkyle ou R24 et R25 forment ensemble avec l'atome d'azote auquel ils sont liés un cycle de 4 à 10 chaînons, monocyclique, bicyclique ou spirocyclique qui peut contenir, outre l'atome d'azote, 0 à 2 hétéroatomes supplémentaires, choisis dans le groupe formé par l'oxygène, l'azote et le soufre, le système cyclique hétérocyclique pouvant en outre être substitué par F, (C₁-C₆)-alkyle, O- (C₁-C₈) -alkyle, oxo, CO(R44), N(R48)CO(C₁-C₆)-alkyle ou N(R49)(R50) ;
o vaut 0, 1, 2, 3, 4 ;
R36 signifie un cycle monocyclique de 5-8 chaînons, qui peut contenir 0-2 hétéroatomes du groupe formé par N, O et S et le cycle de 5-8 chaînons pouvant contenir d'autres substituants tels que F, oxo, O-(C₁-C₆)-alkyle, (C₁-C₆)-alkyle, (C₀-C₂)-alkylénaryle et N (R55) (R56).

13. Procédé pour la préparation des composés selon l'une quelconque des revendications 1 à 12, dont le groupement A-Q est lié au cycle cyclohexylène via un atome d'azote, comprenant les étapes
a) préparation d'un composé de formule II par transformation de cyclohexadione avec du 1,2-éthylèneglycol 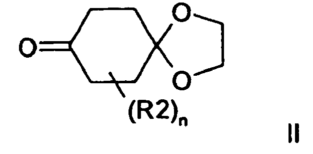
b) transformation du composé de formule II avec 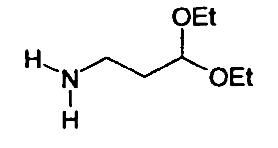 en un composé de formule III 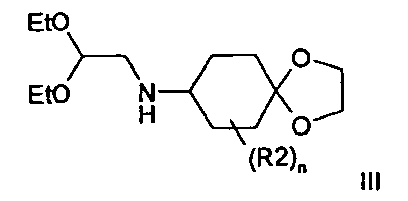
c) couplage du composé de III avec une amine primaire de formule 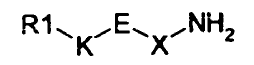 et déprotection consécutive de l'acétal, où on obtient, avec une cyclisation simultanée, un composé de formule IV 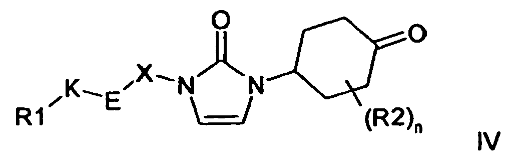
d) transformation avec une amine secondaire, où on obtient des composés de formule I dont le groupement A-Q est lié au cycle cyclohexylène via un atome d'azote

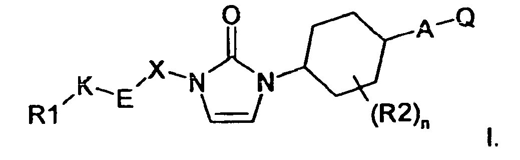

14. Médicament contenant un ou plusieurs des composés selon l'une quelconque des revendications 1 à 12.

15. Médicament contenant un ou plusieurs des composés selon l'une quelconque des revendications 1 à 12 et une ou plusieurs substances actives anorexigènes.

16. Composés de formule I selon l'une quelconque des revendications 1 à 12 pour une utilisation comme médicament destiné à la prophylaxie ou au traitement de l'obésité.

17. Composés de formule I selon l'une quelconque des revendications 1 à 12 pour une utilisation comme médicament destiné à la prophylaxie ou au traitement du diabète de type II.

18. Composés de formule I selon l'une quelconque des revendications 1 à 12 en combinaison avec au moins une autre substance active anorexigène pour une utilisation comme médicament destiné à la prophylaxie ou au traitement de l'obésité.

19. Composés de formule I selon l'une quelconque des revendications 1 à 12 en combinaison avec au moins une autre substance active anorexigène pour une utilisation comme médicament destiné à la prophylaxie ou au traitement du diabète de type II.

20. Procédé pour la préparation d'un médicament contenant un ou plusieurs des composés de formule I selon l'une quelconque des revendications 1 à 12, **caractérisé en ce que** la substance active est mélangée avec un support pharmaceutiquement approprié et ce mélange est amené dans une forme appropriée pour l'administration.

21. Utilisation des composés de formule I selon l'une quelconque des revendications 1 à 12 pour la préparation d'un médicament destiné à la réduction pondérale chez les mammifères.

22. Utilisation des composés de formule I selon l'une quelconque des revendications 1 à 12 pour la préparation d'un médicament destiné à la prophylaxie ou au traitement de l'obésité.

23. Utilisation des composés de formule I selon l'une quelconque des revendications 1 à 12 pour la préparation d'un médicament destiné à la prophylaxie ou au traitement du diabète de type II.
